# EUROPEAN PATENT APPLICATION

(11) **EP 2 601 922 A1**
(43) Date of publication of application: **12.06.2013**
(21) Application number: 11814477.3
(22) Date of filing: 25.07.2011
(51) Int. Cl.: A61F 13/49, A61F 13/15, A61F 13/53

(54) **ABSORBENT PANTS**

(30) Priority: 16.09.2010 JP 2010207778; 03.08.2010 JP 2010174632
(71) Applicant: Kao Corporation, Chuo-ku Tokyo 103-8210 (JP)
(72) Inventor: SASAKI, Jun, Haga-gun Tochigi 321-3497 (JP); OKUDA, Yasuyuki, Haga-gun Tochigi 321-3497 (JP)
(74) Representative: Vossius & Partner
(86) International application number: PCT/JP2011/066835
(87) International publication number: WO 2012/017847

(57) **Abstract**

A diaper (1A) of the invention includes a front panel (2A) a rear panel (2B), an absorbent assembly (3) fixed to the front and the rear panel to bridge them, and a standing gather (4) along each lateral side portion (3c, 3c) of the absorbent assembly (3). The absorbent assembly (3) includes a vertically oblong absorbent member (33). The absorbent member (33) has a pair of fiber-free regions (34, 34) extending in its longitudinal direction. The fiber-free regions (34, 34) each straddle the front panel (2A) and the rear panel (2B).

## Description

### Technical Field

The present invention relates to a pull-on absorbent article, such as a disposable diaper.

### Background Art

A pull-on absorbent article is known, which includes an hourglass-shaped outer cover having a front portion, a crotch portion, and a rear portion adapted to be worn about the front side, the crotch, and the rear side, respectively, of a wearer and an absorbent assembly fixed to the inner side of the outer cover, with both the lateral side edge portions of the outer cover in the front portion and those in the rear portion being joined together to form a waist opening and a pair of leg openings.
Continuous production of such pull-on absorbent articles generally includes the steps of making through-holes or cutouts in a continuous length of outer cover to form leg openings and removing the unnecessary parts (trimmings).

Another type of conventional pull-on absorbent articles is also known in which the outer cover is separated into a front side outer cover to be worn around the front side of a wearer and a rear side outer cover to be worn around the rear side of the wearer, and the absorbent assembly is fixed to bridge the front side outer cover and the rear side outer cover, with both lateral side edge portions of the front side outer cover and those of the rear side outer cover being joined together. An example of this type of pull-on absorbent articles is the absorbent article disclosed in patent literature 1 below, which includes a chassis having a front torso surrounding region and a rear torso surrounding region and an absorbent member straddling the front and the rear torso surrounding region. Another example is the disposable pull-on diaper disclosed in patent literature 2 below, which includes a cylindrical torso surrounding portion formed of a front outer sheet and a rear outer sheet and an inner assembly containing an absorbent core having constricted portions, the inner assembly being fixed to connect the inner side of the front outer sheet and the inner side of the rear outer sheet.

### Citation List

### Patent literature

Patent literature 1: WO2008/06927
Patent literature 2: JP 2008-253285A

### Summary of Invention

A pull-on absorbent article of the type in which the outer cover is separated into two parts: a front outer cover worn around the front side of a wearer and a rear outer cover worn around the back side of the wearer, is advantageous in that the need to trim a continuous length of outer cover may be eliminated, or the size of cutouts may be reduced. This advantage leads to reduction in production and equipment cost and also environmental friendliness. However, such a pull-on absorbent article having two separate outer covers lacks portions covering leg circumferences and therefore has a weakness against leakage from the absorbent assembly, compared with the pull-on absorbent articles having an hourglass-shaped outer cover and an absorbent assembly as discussed above.

According to patent literature 1, the absorbent member has a pair of low-rigidity side portions in substantially parallel relation. Because each of the low-rigidity side is not so long as to straddle the front torso surrounding region and the rear torso surrounding region, the absorbent member does not form a pocket shape while worn and thus fails to prevent leakage.
The disposable pull-on diaper of patent literature 2 has an hourglass-shaped absorbent core for the purpose of improving the crotch appearance and wearer comfort. In this case, too, the absorbent core fails to form a pocket while worn, the feeling of insecurity about leakage from the crotch portion is not removed, and leakage is not prevented.

The invention relates to a pull-on absorbent article which is of the type wherein the outer cover is separated into a front panel adapted to be worn around the front of a wearer and a rear panel adapted to be worn around the rear of the wearer and yet provides protection against leakage from the absorbent assembly without causing the absorbent assembly to hinder the walking motion of a wearer while worn.

The invention provides a pull-on absorbent article including a laterally oblong front panel adapted to be worn around a wearer's front side, a laterally oblong rear panel adapted to be worn around the wearer's rear side, an absorbent assembly fixed to the front and the rear panel to bridge them, and a standing gather disposed along each lateral side of the absorbent assembly. The absorbent assembly includes a vertically oblong absorbent member. The absorbent member has a pair of fiber-free regions extending in its longitudinal direction. Each of the fiber-free regions straddles the front panel and the rear panel.

The invention provides the following pull-on absorbent articles.
[1] A pull-on absorbent article comprising a laterally oblong front panel adapted to be worn around a wearer's front side, a laterally oblong rear panel adapted to be worn around the wearer's rear side, an absorbent assembly fixed to the front and the rear panel to bridge them, and a standing gather disposed along each lateral side portion of the absorbent assembly, the absorbent assembly comprising a vertically oblong absorbent member, the absorbent member having a pair of fiber-free regions extending in its longitudinal direction, each of the fiber-free regions straddling the front panel and the rear panel.
[2] The pull-on absorbent article set forth in [1], wherein the front panel has a front panel main portion and a front panel extension portion extending downward from the lower edge of the front panel main portion while worn, the rear panel has a rear panel main portion and a rear panel extension portion extending downward from the lower edge of the rear panel main portion while worn, and each fiber-free region extends with its vertical front end portion overlapping the front panel extension portion and its vertical rear end portion overlapping the rear panel extension portion.
[3] The pull-on absorbent article set forth in [2], wherein each fiber-free region extends from the position where the vertical front end portion thereof overlaps the front panel extension portion to the position where the vertical rear end portion thereof overlaps the rear panel extension portion.
[4] The pull-on absorbent article set forth in [2] or [3], wherein each of the fiber-free regions extends along the corresponding lateral side edge portion of the absorbent member from the position where the vertical front end portion thereof overlaps the front panel extension portion to the position where the vertical rear end portion thereof overlaps the rear panel extension portion.
[5] The pull-on absorbent article set forth in any one of [2] to [4], wherein the front panel and the rear panel each have a plurality of elastic members disposed vertically spacedly in their laterally stretched state, the elastic members being not disposed in the region where the vertical front end portion of each fiber-free region overlaps the front panel extension portion and the region where the vertical rear end portion of each fiber-free region overlaps the rear panel extension portion.
[6] The pull-on absorbent article set forth in [5], wherein the front panel extension portion and the rear panel extension portion each have a plurality of elastic members disposed vertically spacedly in their laterally stretched state.
[7] The pull-on absorbent article set forth in [6], wherein each of the front panel and the rear panel has an elasticized waist region along the edge of the waist opening, an elasticized below-waist region, and an elasticized extension region in the front or the rear panel extension portion, the elasticized below-waist region being located between the elasticized waist region and the front or the rear panel extension portion.
[8] The pull-on absorbent article set forth in any one of [1] to [7], wherein the standing gather comprises a first standing gather extending laterally inward from the lateral side portion of the absorbent assembly and a second standing gather extending laterally outward from the lateral side portion of the absorbent assembly.
[9] The pull-on absorbent article set forth in any one of [1] to [8], wherein the absorbent member has an elastic member disposed in its vertically stretched state along each lateral side edge portion thereof.
[10] The pull-on absorbent article set forth in any one of [1] to [8], wherein the absorbent member has a single layer structure.
[11] The pull-on absorbent article set forth in any one of [2] to [10], wherein the absorbent member has a high basis weight region, the high basis weight region straddling the front panel extension portion and the rear panel extension portion.
[12] The pull-on absorbent article set forth in [11], wherein the absorbent member comprises an absorbent core, the absorbent core comprising an upper absorbent layer and a lower absorbent layer larger than the upper absorbent layer, the high basis weight region being the result of stacking the upper absorbent layer on the skin facing side of the lower absorbent layer.
[13] The pull-on absorbent article set forth in any one of [1] to [12], wherein each fiber-free region has a closed vertical front end portion in the front portion and a non-closed vertical rear end in the rear portion.
[14] The pull-on absorbent article set forth in [13], wherein each fiber-free region has a closed vertical front end portion in the front portion and a non-closed vertical rear end that is open along the lateral side edge of the absorbent core.
[15] The pull-on absorbent article set forth in any one of [1] to [14], wherein the absorbent member has a central fiber-free region at the middle between the pair of fiber-free regions, the central fiber-free region extending with its front end portion being located beyond the front end portion of each fiber-free region toward the front portion, and each fiber-free region extending with its rear end portion being located beyond the rear end portion of the central fiber-free region toward the rear portion.
[16] The pull-on absorbent article set forth in any one of [1] to [15], wherein the central fiber-free region extends with its vertical front end portion reaching the front panel main portion.
[17] The pull-on absorbent article set forth in any one of [1] to [16], wherein the central fiber-free region extends with its vertical front end portion terminating in the elasticized below-waist region of the front portion.
[18] The pull-on absorbent article set forth in any one of [15] to [17], wherein the absorbent member has a region in which the central fiber-free region and the pair of fire-free regions are in juxtaposition in the lateral direction.
[19] The pull-on absorbent article set forth in any one of [1] to [18], wherein each of the pair of fiber-free regions is devoid of pulp fiber and a particulate absorbent polymer which constitute the absorbent core of the absorbent member.
[20] The pull-on absorbent article set forth in any one of [1] to [19], wherein each of the pair of fiber-free regions contains a total of up to 30 g/m² of pulp fiber and a particulate absorbent polymer which constitute the absorbent core of the absorbent member.
[21] The pull-on absorbent article set forth in any one of [1] to [20], wherein the absorbent member comprises a vertically oblong absorbent core containing an absorbent polymer and a core wrap sheet covering the upper and the lower surface of the absorbent core, the absorbent core having a central fiber-free region at the middle between the pair of fiber-free regions, the part of the core wrap sheet overlying the absorbent core and the part of the core wrap sheet underlying the absorbent core being bonded to each other in the pair of fiber-free regions, the part of the core wrap sheet overlying the absorbent core and the member lying right under that part of the core wrap sheet being not bonded to each other in the central fiber-free region.
[22] The pull-on absorbent article set forth in any one of [1] or [21], wherein the absorbent member has a central absorbent portion extending in the longitudinal direction of the absorbent core and a pair of side absorbent portions each located on each lateral side of the central absorbent portion, and the absorbent polymer is present in the central absorbent portion in a higher basis weight than in each of side absorbent portions.
[23] The pull-on absorbent article set forth in [21] or [22], wherein the absorbent member has a multi-layered structure comprising an upper absorbent layer and a lower absorbent layer, and the absorbent polymer is present in the upper absorbent layer in a higher basis weight than in the lower absorbent layer.

### Brief Description of Drawings

[Fig. 1] Fig. 1(a), Fig. 1(b), and Fig. 1(c) each illustrate a disposable pull-on diaper according to a first embodiment of the invention while in use (while worn), of which Fig. 1(a) is a view from the front of a wearer, Fig. 1(b) is a side view, and Fig. 1(c) is a back view.
[Fig. 2] Fig. 2 is a plan of the disposable pull-on diaper shown in Figs. 1(a) to 1(c) in its flat-out, uncontracted state, seen from the topsheet side. As used herein, the term "flat-out, uncontracted state" means a state in which a pull-on absorbent article is opened by tearing the side seals apart and with every elastic member straightened up to its design dimension (the dimension of an article in a flat-out configuration with any influences of elastic members eliminated).
[Fig. 3] Fig. 3 is an enlarged plan of an essential part of the disposable pull-on diaper of Fig. 1, showing the relation between the absorbent member and the front panel.
[Fig. 4] Fig. 4 is a cross-section taken along line X1-X1 of Fig. 2.
[Fig. 5] Fig. 5 is a plan of a disposable pull-on diaper according to a second embodiment of the invention in its flat-out, uncontracted state, seen from the topsheet side.
[Fig. 6] Fig. 6 is a plan of a disposable pull-on diaper according to a third embodiment of the invention in its flat-out, uncontracted state, seen from the topsheet side.
[Fig. 7] Fig. 7 is a cross-section taken along line X2-X2 of Fig. 6.
[Fig. 8] Fig. 8 is a cross-section of a disposable pull-on diaper according to another embodiment of the invention, corresponding to Fig. 7.
[Fig. 9] Fig. 9 is a perspective of a disposable pull-on diaper according to still another embodiment of the invention.
[Fig. 10] Fig. 10 is a plan of the disposable pull-on diaper shown in Fig. 9 in its flat-out, uncontracted state with a part cut away, seen from the skin facing side. As used herein, the term "flat-out, uncontracted state" means a state in which a pull-on absorbent article is opened by tearing the side seals apart and with every elastic member straightened up to its design dimension (the dimension of an article in a flat-out configuration with any influences of elastic members eliminated).
[Fig. 11] Fig. 11 is a cross-section taken along line III-III of Fig. 10.
[Fig. 12] Fig. 12(a) and Fig. 12(b) are each a schematic transverse cross-section of the absorbent core of the disposable diaper while worn, of which Fig. 12(a) is a cross-section taken at the position indicated by arrow PI in Fig. 10, and Fig. 12(b) is a cross-section taken at the position indicated by arrow P2 in Fig. 10.
[Fig. 13] Fig. 13 is a perspective view illustrating a preferred embodiment of the production of a disposable diaper according to the invention.

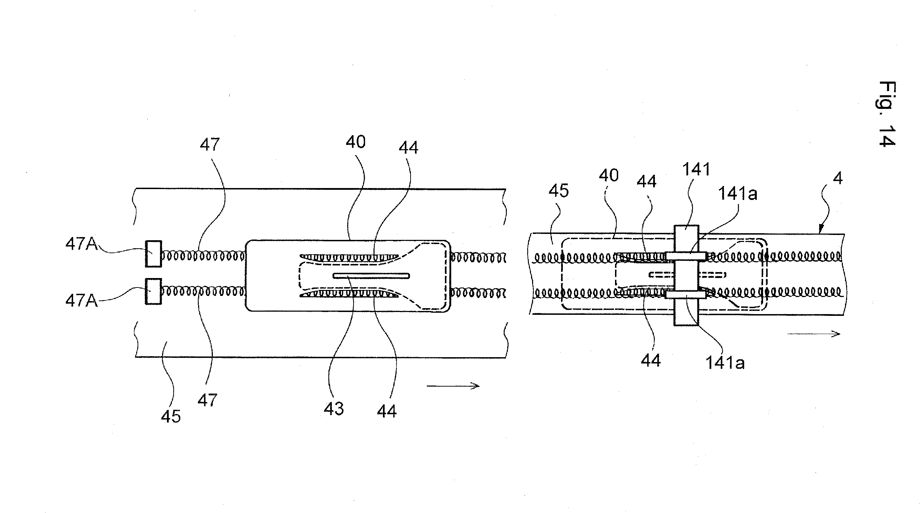

[Fig. 14] Fig. 14 is a plan view illustrating a preferred embodiment of the production of a disposable diaper according to the invention.
[Fig. 15] Fig. 15 is a transverse cross-section of the absorbent member used in a yet another embodiment of the invention.

### Description of Embodiments

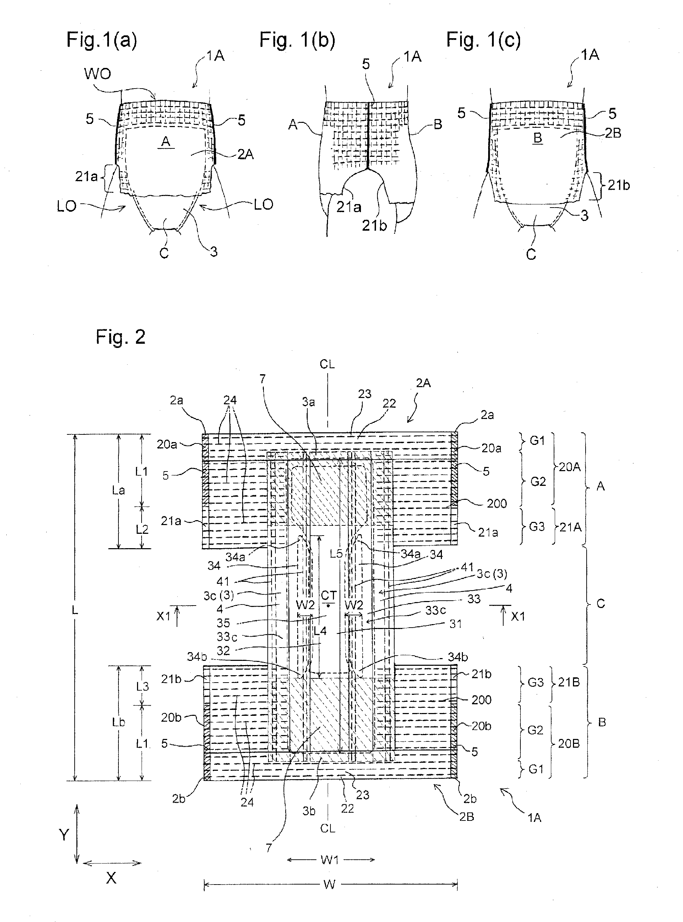

The pull-on absorbent article of the invention will be described based on its first preferred embodiment with reference to Figs. 1 through 4.

As shown in Figs. 1 and 2, a disposable pull-on diaper 1A of the first embodiment (hereinafter "diaper 1A") includes a laterally oblong rectangular front panel 2A adapted to be worn about the front side of a wearer, a laterally oblong rectangular rear panel 2B adapted to be worn about the rear side of a wearer, an absorbent assembly 3 fixed to the front panel 2A and the rear panel 2B to bridge them, and a standing gather 4 provided along both lateral side portions 3c, 3c of the absorbent assembly 3. In the first embodiment, part of the lateral side edge portions 2a and 2a of the front panel 2A and corresponding part of the lateral side edge portions 2b and 2b of the rear panel 2B are joined together to form a waist opening WO and a pair of leg openings LO, LO.
As shown in Figs. 1 and 2, the diaper 1A is bilaterally symmetrical about the centerline CL. Therefore, the bilaterally symmetrical part of the diaper 1A will be described chiefly with reference to the right-hand side.

In more detail, the diaper 1A of the first embodiment has, in its uncontracted state (see Fig. 2), a front portion A and a rear portion B adapted to be worn about the front side and the rear side, respectively, of a wearer while worn arranged in a vertical direction (hereinafter "direction Y"; direction Y is defined to be a direction parallel to the centerline CL) and a crotch portion C located between the front portion A and the rear portion B and adapted to be worn about the crotch of a wearer while worn. The lateral or transverse direction of the diaper 1A is a direction perpendicular to the vertical direction (direction Y) (hereinafter "direction X").

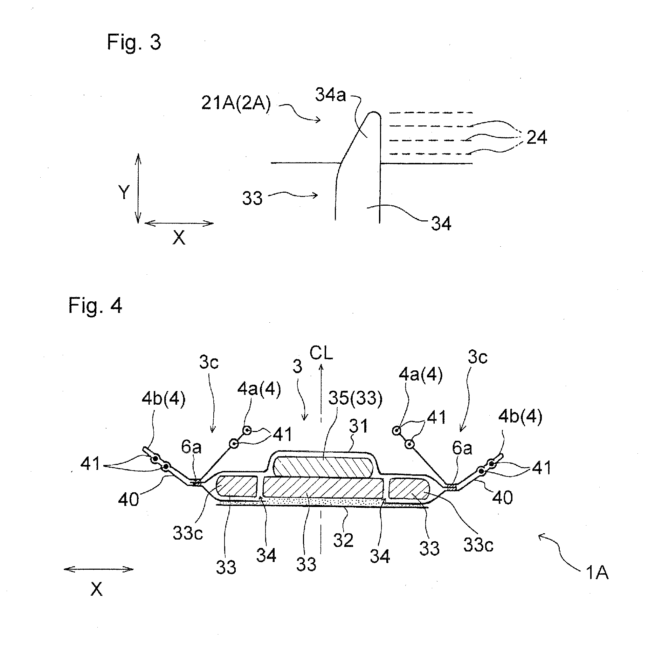

As shown in Figs. 2 and 4, the absorbent assembly 3 of the diaper 1A has a vertically elongated absorbent member 33. The absorbent member 33 has a pair of fiber-free regions 34, 34 each extending in the longitudinal direction of the absorbent member 33 along each lateral side edge portion 33c of the absorbent member 33. In detail, as shown in Figs. 2 and 4, the absorbent assembly 3 includes a liquid permeable topsheet 31, a liquid impermeable or water repellent backsheet 32, and a liquid retentive absorbent member 33 interposed between the sheets 31 and 32 and, as shown in Fig. 2, has a vertically (direction Y) elongated rectangular shape. The absorbent member 33 includes an absorbent core formed of an aggregate of fibers, such as pulp fiber, with or without a particulate absorbent polymer held therein, and a core wrap sheet (unshown) covering the absorbent core. The absorbent member 33 also has a vertically (direction Y) elongated rectangular shape. As shown in Fig. 2, the absorbent core of the absorbent member 33 has a pair of fiber-free regions 34 that are devoid of pulp fibers, absorbent polymer particles, and the like. As used herein, the term "fiber-free region" means not only a region having no material, such as pulp fibers and absorbent polymer particles, but a region having a small basis weight, e.g., up to about 30 g/m², of pulp fibers, absorbent polymer particles, and the like because the latter region produces the same effects as by the former region. It should be noted that the basis weight of the fiber-free region is preferably 20 g/m² or less to ensure the effects.

The basis weight above referred to can be measured, for example, as follows. The absorbent member 33 of the absorbent assembly 3 is taken out of the diaper 1A. The core wrap sheet is removed from the absorbent member 33 to expose the absorbent core. A portion having a prescribed width including a fiber-free region 34 is cut out of the absorbent core to prepare a cut sample of the fiber-free region. The weight of the cut sample is measured using an electronic balance having a minimum readability of 1 mg and converted to a basis weight. To increase the precision of basis weight measurement, it is advisable to prepare at least five cut samples from a plurality of diapers 1A to obtain an average of five or more measurements.

As shown in Fig. 2, the absorbent member 33 has a high basis weight region 35. In detail, as shown in Fig. 2, the absorbent core constructing the absorbent member 33 of the first embodiment is composed of a T-shaped upper absorbent layer and a vertically (direction Y) elongated rectangular lower absorbent layer larger than and longer in the vertical direction (direction Y) than the upper absorbent layer. The high basis weight region 35 is provided in the shape of the letter "T" as a result of stacking the upper absorbent layer on the skin facing surface of an area extending from the front end portion to the crotch portion C of the lower absorbent layer. Each of the paired fiber-free regions 34 provided in the absorbent member 33 is located, as shown in Fig. 2, between an X-direction edge of the absorbent member 33 and the corresponding X-direction distal edge of the high basis weight region 35 and extends along the corresponding lateral side edge portion 33c of the absorbent member 33 and also along the corresponding lateral side of the T-shaped high basis weight region 35. The T-shaped configuration of the upper absorbent layer as in the present embodiment achieves efficient arrangement of the absorbent member 33 in the front and the crotch portion having a high likelihood of being used in practice, thereby to ensure prevention of side leakage from the crotch portion C.

The basis weight of the high basis weight region 35 is higher than the region other than the high basis weight region 35 and the fiber-free regions 34 and preferably ranges from 100 to 400 g/m². The basis weight of the region other than the high basis weight region 35 and the fiber-free regions 34 is preferably 50 to 200 g/m². The basis weights of these regions may be determined by the same method as described above.

As shown in Fig. 2, the front panel 2A of the diaper 1A according to the first embodiment has a front panel main portion 20A and a front panel extension portion 21A extending downward from the lower edge 200 of the front panel main portion 20A while worn. As used herein, the term "downward" means toward the transverse centerline CT (see Fig. 2) of the diaper 1A. Specifically, the front panel main portion 20A is a rectangle elongated in the diaper transverse direction (direction X) in its uncontracted state (see Fig. 2) and has a laterally opposite pair of side edge portions 20a, 20a extending in the vertical direction (direction Y). The front panel extension portion 21A extends downward from the lower edge 200 of the front panel main portion 20A. The front panel extension portion 21A is a rectangle elongated in the diaper transverse direction (direction X) in its uncontracted state (see Fig. 2) and has a laterally opposite pair of side edge portions 21a, 21a extending in the vertical direction (direction Y).

As shown in Fig. 2, the rear panel 2B of the diaper 1A according to the first embodiment has a rear panel main portion 20B and a rear panel extension portion 21B extending downward from the lower edge 200 of the rear panel main portion 20B while worn. As used herein, the term "downward" means, similarly to the front panel 2A, toward the transverse centerline CT (see Fig. 2) of the diaper 1A. Specifically, the rear panel main portion 20B is a rectangle elongated in the diaper transverse direction (direction X) in its uncontracted state (see Fig. 2) and has a laterally opposite pair of side edge portions 20b, 20b extending in the vertical direction (direction Y). The rear panel extension portion 21B extends downward from the lower edge 200 of the rear panel main portion 20B. The rear panel extension portion 21B is a rectangle elongated in the diaper transverse direction (direction X) in its uncontracted state (see Fig. 2) and has a laterally opposite pair of side edge portions 21b, 21b extending in the vertical direction (direction Y).

In the diaper 1A of the first embodiment, a part each of the side edge portions 2a, 2a of the front panel 2A and a corresponding part each of the side edge portions 2b, 2b of the rear panel 2B are joined together, thereby to form a waist opening WO and a pair of leg openings LO, LO. More specifically, as shown in Fig. 2, the side edge portions 20a. 20a of the front panel main portion 20A and the side edge portions 20b, 20b of the rear panel main portion 20B are joined together over their whole length, whereas the side edge portions 21a, 21a of the front panel extension portion 21A and the side edge portions 21b, 21 b of the rear panel extension portion 21 B are not bonded together. The side edge portions 20a, 20a of the front panel main portion 20A and the side edge portions 20b, 20b of the rear panel main portion 20B are thus joined together to form a pair of opposite side seals 5, 5, the waist opening WO, and a pair of leg openings LO, LO. Joining the front and the rear panel is achieved by, for example, heat sealing, high frequency sealing, ultrasonic sealing, or adhesive application.

The length La of the front panel 2A is preferably 10% to 40%, more preferably 15% to 35%, of the total diaper length L. The length Lb of the rear panel 2B is preferably 20% to 40%, more preferably 25% to 35%, of the total diaper length L. The length L2 of the front panel extension portion 21A is preferably 5% to 100%, more preferably 25% to 70%, of the length L1 of each side seal 5 (i.e., the length of the front panel main portion 20A). The length L2 of the front panel extension portion 21A is preferably 5% to 50%, more preferably 10% to 40%, of the length La of the front panel 2A. The length L3 of the rear panel extension portion 21 B is preferably 5% to 100%, more preferably 25% to 70%, of the length L1 of each side seal 5 (i.e., the length of the rear panel main portion 20B). The length L3 of the rear panel extension portion 21 B is preferably 10% to 50%, more preferably 20% to 40%, of the length Lb of the rear panel 2B. For use by children, the extension lengths L2 and L3 of the front and the rear panel extension portion 21A and 21B, respectively, are preferably 10 to 70 mm, more preferably 20 to 60 mm. For use by adults, the extension lengths L2 and L3 are preferably 15 to 110 mm, more preferably 30 to 90 mm. It is preferred that the extension length L2 of the front panel extension portion 21A be smaller than the extension length L3 of the rear panel extension portion 21B so that the front and the back of the diaper 1A as a garment may be visually distinguished when fitting to a wearer.

As shown in Fig. 2, the front panel 2A and the rear panel 2B each have a plurality of elastic members 24 vertically (direction Y) spacedly disposed in their laterally (direction X) stretched state. That is, as shown in Fig. 2, the front panel 2A of the diaper 1A has the main portion 20A and the extension portion 21A, and the rear panel 2B of the diaper 1A has the main portion 20B and the extension portion 21 B as earlier stated, and each of the front panel main portion 20A and the rear panel main portion 20B as well as each of the front panel extension portion 21A and the rear panel extension portion 21B has a plurality of elastic members 24 vertically (direction Y) spacedly disposed in their laterally (direction X) stretched state. As shown in Fig. 2, the front panel 2A (the main portion 20A, the extension region 21A) and the rear panel 2B (the main portion 20B, the extension region 21 B) of the diaper 1A each include an outer sheet 22 defining the outer surface of the diaper, an inner sheet 23 disposed on the inner side of the outer sheet 22, and a plurality of elastic members 24 disposed between the sheets 22 and 23 in their laterally (direction X) stretched state. The plurality of elastic members 24 are arranged vertically (direction Y) spacedly to form, upon contraction, an elasticized waist region G1 along the edge of the waist opening WO, an elasticized below-waist region G2, and an elasticized extension region G3 in each of the front panel extension portion 21A and the rear panel extension portion 21B. As shown in Fig. 2, the outer sheet 22 of the diaper 1A of the first embodiment extends vertically (direction Y) outward beyond its region where the inner sheet 23 are laid thereon to have the plurality of elastic members 24 of thread form sandwiched therebetween. The extended portion of the outer sheet 22 extending beyond the inner sheet 23 is folded back over the side of the absorbent assembly 3 to cover the corresponding vertical end portion 3a, 3b of the absorbent assembly 3 and fixed to the inner sheet 23 with an unshown adhesive.

As shown in Fig. 2, the elasticized waist region G1 is provided, in each of the front panel 2A and the rear panel 2B, outward from the corresponding longitudinal (direction Y) end 3a, 3b of the absorbent assembly 3. The elasticized below-waist region G2 is provided, in each of the front panel 2A and the rear panel 2B, between the elasticized waist region G1 and the front panel extension portion 21a or the rear panel extension portion 21b. The elasticized extension region G3 is formed in the front panel extension portion 21 a and in the rear panel extension portion 21b.

Every elasticized member 24 forming the elasticized waist region G1 is disposed over the whole width (direction X) of the front panel 2A and the rear panel 2B. In the first embodiment, the elastic members 24 forming the elasticized below-waist region G2 and the elasticized extension region G3 are not disposed in the regions where the absorbent member 33 of the absorbent assembly 3 overlaps the front and the rear panel or, if any, the elasticized member 24 does not function to elasticize that region of the front and rear panel 2A and 2B as a result of, for example, thermally deelasticizing the elastic member 24 or cutting the elastic member 24. In other than the regions where the absorbent assembly 3 and the front and the rear panel overlap, these elastic members 24 extend in the lateral direction (direction X) to elasticize the front panel and the rear panel 2A and 2B.

Because the diaper 1 of the first embodiment has a front extension portion 21A in the front panel 2A as stated, it has a good appearance from the side of the front portion A without looking like loincloth.
Because the elasticized extension region G3 formed in each of the front extension portion 21 A and the rear extension portion 21 B is contracted to a moderate degree by the elastic members 24 disposed therein, when the diaper 1 of the present embodiment is fitted to a wearer, the side edge 21 a of the front extension portion 21 A of the front panel 2A and the side edge 21b of the rear extension portion 21B of the rear panel 2B on each side of the diaper separate from each other toward the front and the rear, respectively, to make the shape of an inverted Y as shown in Fig. 1(b). Therefore, the diaper 1 has a good appearance from the side and looks like providing good protection against leakage.

Because the diaper 1 of the first embodiment has a front extension portion 21A in the front panel 2A as stated, and the front extension portion 21A forms the elasticized extension region G3 that stretches and contracts in the diaper lateral direction, the front extension portion 21A comes into planar contact to the skin to provide coverage on the groins. After urination, the increased weight is supported by the front extension portion 21A, the front panel main portion 20A above the front extension portion 21A, and the rear panel main portion 20B. As a result, the pressure is diffused to prevent leaving red marks around wearer's thighs.

As stated, the diaper 1 of the present embodiment has the rear panel extension portion 21B and the elasticized extension region G3 that stretches and contracts in the diaper lateral direction in the rear panel 2B. As a result, the rear panel extension portion 21B comes into planar contact to the skin to provide coverage on the buttocks, and the diaper of the present embodiment is less likely to cause a part of the wearer's buttocks to be exposed than conventional diapers having the lateral side edge portions of the front panel 2A and those of the rear panel 2B bonded together over their whole length in the vertical direction.

The absorbent assembly 3 is fixed in its region on the side of the longitudinal (direction Y) end portion 3a to the front panel 2A with an adhesive 7 and in a region on the side of the opposite longitudinal (direction Y) end portion 3b to the rear panel 2B with an adhesive 7. In detail, in the front portion A of the diaper 1A of the first embodiment, the adhesive 7 is preferably applied to a region between the laterally (direction X) opposite edges of the lateral side portions 3c, 3c of the absorbent assembly 3 and vertically (direction Y) outward from, and including, an imaginary line connecting the very ends of the vertical front end portions 34a (hereinafter described) of the pair of fiber-free regions 34 formed in the absorbent member 33 as is shown in Fig. 2. From an aesthetic viewpoint, it is more preferred that the adhesive be applied to a region vertically (direction Y) outward from a position located between the imaginary line and the lower edge 200 of the front panel main portion 20A as shown in Fig. 2.

In the rear portion B of the diaper 1A of the first embodiment, the adhesive 7 is preferably applied to a region between the laterally (direction X) opposite edges of the lateral side portions 3c, 3c of the absorbent assembly 3 and vertically (direction Y) outward from, and including, an imaginary line connecting the very ends of the vertical rear end portions 34b (hereinafter described) of the pair of fiber-free regions 34 formed in the absorbent member 33 as shown in Fig. 2. From the standpoint of preventing the wearer's buttocks from being exposed, it is more preferred that the adhesive 7 be applied to a region vertically (direction Y) outward from the imaginary line as shown in Fig. 2.
In the diaper 1A of the first embodiment, the distance from the proximal(in the direction Y) end of the adhesive 7 in the rear portion B to the very ends of the hereinafter described vertical rear end portions 34b of the pair of fiber-free regions 34 is shorter than the distance from the proximal (in the direction Y) end of the adhesive 7 in the front portion A to the very ends of the hereinafter describe vertical front end portions 34a of the pair of fiber-free regions 34.

As shown in Fig. 2, the pair of fiber-free regions 34 formed in the absorbent member 33 straddle the front panel 2A and the rear panel 2B. In the diaper 1A of the first embodiment, the vertical (direction Y) front end portion 34a of each fiber-free region 34 overlaps the front panel extension portion 21A, and the vertical rear end portion 34b of each fiber-free region 34 overlaps the rear panel extension portion 21B. In detail, each of the fiber-free regions 34 of the diaper 1A of the first embodiment extends in the direction Y along the corresponding lateral side edge portion 33c of the absorbent member 33 from the position where the vertical front end portion 34a thereof overlaps the front panel extension portion 21A of the front panel 2A to the position where the vertical rear end portion 34b thereof overlaps the rear panel extension portion 21 B of the rear panel 2B.

The absorbent member 33 preferably has a width W1 in the lateral direction (direction X) ranging from 35% to 65%, more preferably from 40% to 60%, of the width W of the front and the rear panel 2A and 2B in the lateral direction (direction X) in the state shown in Fig. 2. Each fiber-free region 34 preferably has a width W2 in the lateral direction (direction X) ranging from 2% to 30%, more preferably from 5% to 20%, of the width W1 of the absorbent member 33 so that the absorbent member 33 may provide a good fit to the contour of wearer's legs without causing hindrance to the wearer's walking motion, easily form a pocket convex outward, and secure sufficient absorbing performance in its crotch portion C while worn. The width W2 of each fiber-free region 34 is preferably 2 to 30 mm, more preferably 5 to 25 mm, for use by children and 3 to 40 mm, more preferably 5 to 30 mm, for use by adults.
Each fiber-free region 34 preferably has a length L5 in the vertical direction (direction Y) of from 20% to 70%, more preferably 30% to 60%, of the length L4 of the vertical direction (direction Y) of the absorbent member 33.

In the diaper 1A of the first embodiment, the elastic members 24 are not disposed in the region where the vertical front end portion 34a of each fiber-free region 34 overlaps the front panel extension portion 21A and the region where the vertical rear end portion 34b of each fiber-free region 34 overlaps the rear panel extension portion 21B. As used herein, the expression "the elastic members 24 are not disposed" is intended to mean that the elastic members 24 are not at all disposed or, if any, the disposed elastic member 24 is in a state unable to elasticize the front and rear panel 2A and 2B. In the first embodiment, as previously discussed, there are no elastic members 24 forming the elasticized extension region G3 in the area where the absorbent member 33 of the absorbent assembly 3 overlaps the front and the rear panel extension portion or, if any elastic member 24 is disposed there, the elastic member 24 is in a state unable to elasticize the front panel 2A and the rear panel 2B. For example, no elastic members 24 are disposed in the region where the vertical front end portion 34a of each fiber-free region 34 overlaps the front panel extension portion 21A as shown in Fig. 3. In the diaper 1A of the first embodiment, there are no elastic members 24 forming the elasticized extension region G3 in the entire region where the absorbent member 33 overlaps the front panel extension portion 21 A or, if any, they are present in a state unable to elasticize the front panel 2A and the rear panel 2B. However, it is only necessary that such a deelasticized state is secured in the region where the vertical front end portion 34a of each fiber-free region 34 overlaps the front panel extension portion 21A. That is, the elastic member 24 forming the elasticized extension region G3 may be disposed to elasticize in other than the region where the vertical front end portions 34a of the fiber-free regions 34 overlap the front panel extension portion 21 A.

In the diaper 1A of the first embodiment, the high basis weight region 35 of the absorbent member 3 straddles the front panel extension portion 21 A and the rear panel extension portion 21 B as shown in Fig. 2. In detail, the T-shaped high basis weight region 35 lies extending from the front panel main portion 20A of the front panel 2A, through the front panel extension portion 21 A, to the rear panel extension portion 20B of the rear panel 2B as shown in Fig. 2 so that the front panel 2A may have an increased absorption capacity.

In the diaper 1A of the first embodiment, the standing gather 4 provided in each lateral side portion 3c of the absorbent assembly 3 is composed of a first standing gather 4a extending laterally (direction X) inward from the lateral side portion 3c of the absorbent assembly 3 and a second standing gather 4b extending laterally (direction X) outward from the side portion 3c of the absorbent assembly 3. Each of the first and the second standing gather 4a and 4b is formed of a standing gather-forming sheet 40 and a plurality of elastic members 41 disposed in their stretched state in the vertical direction (direction Y in a flat-out, uncontracted state of the pull-on absorbent article) of the standing gather-forming sheet 40. Specifically, in the diaper 1A of the first embodiment, the standing gather-fonning sheet 40 is a single sheet, which is folded in half into a two-ply sheet along a folding line corresponding to the tip of the free edge of the first standing gather 4a. The plurality of elastic members 41 are fixed in their stretched state in the vertical direction (direction Y)with an adhesive between the two plies of the folded sheet to make the first standing gather 4a as shown in Fig. 4.

In the diaper 1A of the first embodiment, the two-ply sheet extends laterally (direction X) outward and is further folded in half along a folding line corresponding to the tip of the free edge of the second standing gather 4b into a four-ply sheet as shown in Fig. 4. A plurality of elastic members 41 are disposed and fixed with an adhesive in their stretched state in the vertical direction (direction Y) between the two-ply panels of the four-ply sheet to form the second standing gather 4b. As shown in Fig. 4, the topsheet 31 of the diaper 1A of the first embodiment is longer than the backsheet 32 in the lateral direction (direction X), and the portion of the topsheet 31 extending beyond each lateral (direction X) side edge of the absorbent member 33 is bonded to the above described two-ply panels at a first linear joint 6a that is provided slightly outward from each lateral (direction X) side edge of the absorbent member 33. As shown in Fig. 4, the edge of the two-ply sheet is inserted between the absorbent member 33 and the backsheet 32 and fixed there with an adhesive. The thus formed first standing gather 4a rises by the contraction of the plurality of elastic members 41 toward the skin of a wearer and laterally (direction X) inward (toward the centerline CL). The thus formed second standing gather 4b rises, by the contraction of the plurality of elastic members 41, toward the wearer's skin and laterally (direction X) outward (away from the centerline CL).

The first linear bond 6a extends along each side edge portion of the rectangular absorbent assembly 3 over the whole length of the absorbent assembly 3 in the vertical direction (direction X). The first linear bond 6a is preferably a continuous straight line but may be a dotted line. The first linear bond 6a may be formed by various known methods, such as heat sealing, ultrasonic sealing, high frequency sealing, or application of an adhesive.

In the diaper 1A of the first embodiment, the plurality of elastic members 41 forming the first standing gather 4a and the second standing gather 4b extend with their front ends terminating in the area where the adhesive 7 is applied to fix the absorbent assembly 3 and the front panel 2A and with their rear ends terminating in the area where the adhesive 7 is applied to fix the absorbent assembly 4 to the rear panel 2B. Providing the plurality of elastic members 41 to form the first and the second standing gather 4a and 4b helps the absorbent member 33 of the absorbent assembly 3 to form an outward convex pocket while the diaper is worn.

Materials making up the disposable pull-on diaper 1A of the first embodiment will then be described.
The outer and the inner sheet 22 and 23 making up the front panel 2A and the rear panel 2B may be of any materials that have been commonly used in absorbent articles, such as disposable diapers. For example, water repellent nonwoven fabrics may be used to form the outer and the inner sheet 22 and 23.

The topsheet 31 and the backsheet 32 constituting the absorbent assembly 3 may be of any materials that have been commonly used in absorbent articles, such as disposable diapers. For example, the topsheet 31 may be formed of water-wettable and liquid permeable nonwoven fabric, and the backsheet 32 may be formed of liquid impermeable or water repellent resin film or a laminate of such resin film and nonwoven fabric.

The standing gather-forming sheet 40 used to form the standing gather 4 may be of any materials that have been commonly used in absorbent articles, such as disposable diapers. For example, a liquid resistant or water repellent, air-permeable stretch film, nonwoven fabric, woven fabric or laminates thereof may be used as the standing gather-forming sheet 40.

The elastic member 24 disposed in the front and the rear panel 2A and 2B and the elastic member 41 disposed in the standing gather-forming sheet 40 (including a first elastic member 41a) may be elastic threads made of natural rubber, polyurethane, a styrene-isoprene copolymer, a styrene-butadiene copolymer, or an ethylene-α-olefin copolymer (e.g., ethyl acrylate-ethylene copolymer) may be used.

The disposable pull-on diaper 1A according to the first embodiment of the invention produces the following effects and advantages.
As shown in Figs. 1 and 2, the diaper 1A of the first embodiment includes the front panel 2A, the rear panel 2B, and the absorbent assembly 3, and the absorbent member 33 of the absorbent assembly 3 has a pair of fiber-free regions 34 extending along both lateral side portions 33c, 33c of the absorbent member 33 to reach the front panel 2A and the rear panel 2B. As previously discussed, in the case when rectangular sheets are used as the front and the rear panel 2A and 2B, the diaper lacks portions covering leg circumferences particularly in the crotch portion C. This evokes a feeling of insecurity about leakage from the crotch portion C or leakage due to the gap formed around the crotch portion C, which has been a great problem to be solved. To solve this problem, it is important how to improve the fit of the crotch portion C of the absorbent member 33 for a wearer. When the diaper 1A having a pair of fiber-free regions 34 arranged as in the first embodiment is worn, formation of the standing gathers 4 by the contraction of the elastic members 41 is accompanied by bending of the absorbent member 33 in the crotch portion C along the fiber-free regions 34 to form a pocket that is convex downward. In particular, the configuration of the fiber-free regions 34 reaching the front panel and the rear panel 2A and 2B and of the absorbent member 33 being fixed to neither the front panel 2A or the rear panel 2B in the region where the longitudinal end portions of each fiber-free region overlaps the front panel 2A or the rear panel 2B makes it easier for the absorbent member 33 to bend over the whole length of the crotch portion C, whereby the fit on the skin of a wearer is further improved. Thus, although the diaper 1A of the first embodiment is a pull-on absorbent article of the type in which the outer cover is separated into a front panel adapted to be worn around the front of a wearer and a rear panel adapted to be worn around the rear of the wearer, it securely prevents leakage from the absorbent assembly 3 without causing the absorbent assembly to hinder the walking motion of a wearer while worn because of the pocket formed by the absorbent member 33 of the absorbent assembly.

As shown in Fig. 3, because the diaper 1A of the first embodiment has no elastic members 24 disposed in the region where the vertical front end portion 34a of each fiber-free region 34 overlaps the front panel extension portion 21A and the region where the vertical rear end portion 4b of each fiber-free region 34 overlaps the rear panel extension portion 21 B and also because the elastic members 24 exist between the laterally (direction X) distal edge of each fiber-free region 34 and the corresponding lateral (direction X) side edge of the front panel 2A and of the rear panel 2B, the contractive force of these elastic members 24 does not contract the fiber-free regions 34 but pulls the fiber-free regions 34 laterally (direction X) outward. This synergistically results in the effect that the shape of the non-elasticized regions which act as bending-inducing lines is maintained more markedly to further facilitate formation of a downward convex pocket in the crotch portion C.

As shown in Figs. 2 and 4, since the diaper 1A of the first embodiment has the first standing gather 4a and the second standing gather 4b extending laterally (direction X) inward and outward, respectively, from each side portion 3c of the absorbent assembly 3, it provides improved protection against leakage from the absorbent assembly 3 while worn. Additionally, the contractive force of the elastic members 41 forming the first and the second standing gather 4a and 4b reliably makes the absorbent member 33 bend in the crotch portion C along the fiber-free regions 34 and easily form a downward convex pocket.

As shown in Figs. 2 and 4, the absorbent member 3 of the diaper 1A of the first embodiment has the high basis weight region 35 straddling the front panel extension portion 21A and the rear panel extension portion 21B (i.e., extending over the whole length of the crotch portion C). Therefore, it is possible to certainly increase the absorption capacity of the absorbent member in the crotch portion C, which is likely to have a leakage problem, of the diaper 1A according to the first embodiment. Thus, reduction of leakage from the absorbent assembly 3 is further ensured. Furthermore, a marked difference in basis weight is produced between the high basis weight region 35 and the lateral side portions 33c of the absorbent member 33, which are located laterally (direction X) outward from the fiber-free regions 34. As a result, the absorbent member 33 bends more easily along the proximal edge (the edge closer to the high basis weight region 35) of each fiber-free region 34 to form a downward convex pocket more easily.

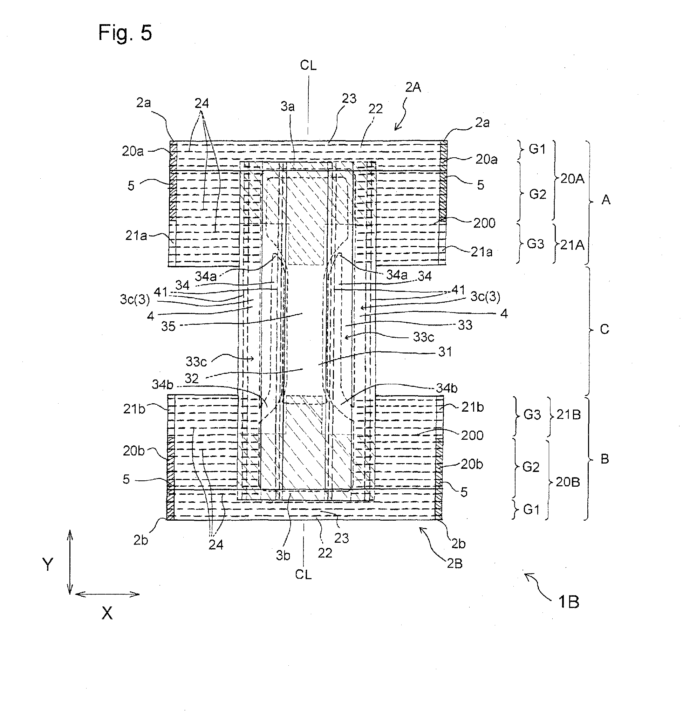

A disposable pull-on diaper according to a second embodiment of the invention will then be described with reference to Fig. 5.
The disposable pull-on diaper 1B according to the second embodiment (hereinafter "diaper 1B") will be described with respect to the differences from the diaper 1A of the first embodiment. The diaper 1B is otherwise equal to the diaper 1A so that the description of the diaper 1A will be applied as appropriate for the rest.

The absorbent assembly 3 of the diaper 1B of the second embodiment is bonded on its vertical (direction Y) front end portion 3a to the front panel 2A with an adhesive 7 applied in the shape of a T and on its vertical (direction Y) rear end portion 3b to the rear panel 2B with an adhesive 7 applied in the shape of an inverted T. In detail, as shown in Fig. 5, in the front portion A of the diaper 1B of the second embodiment, the adhesive 7 is applied to a region of the elasticized below-waist region G2 between the laterally opposite edges of the lateral side portions 3c, 3c of the absorbent assembly 3 and to a region of the elasticized extension region G3 between the pair of fiber-free regions 34. Similarly, in the rear panel B of the diaper 1 B of the second embodiment, the adhesive 7 is applied to a region of the elasticized below-waist region G2 between the laterally opposite edges of the lateral side portions 3c, 3c of the absorbent assembly 3 and to a region of the elasticized extension region G3 between the pair of fiber-free regions 34 as shown in Fig. 5.

The pair of fiber-free regions 34 of the diaper 1B of the second embodiment are different in shape from those of the diaper 1A as shown in Fig. 5. Specifically, as shown in Fig. 5, each fiber-free region 34 formed in the absorbent member 33 of the diaper 1B has a closed vertical front end portion 34a in the front portion A, which is the same as in the diaper 1A, but the vertical rear end portion 34b of each fiber-free region 34 in the rear portion B is not closed but open along the lateral side edge of the absorbent core. In the diaper 1B of the second embodiment, the elastic members 24 are not disposed in either a region where the vertical front end portion 34a of each fiber-free region 34 overlaps the front panel extension portion 21A or a region where the vertical rear end portion of each fiber-free region 34 is open in the rear portion B.

Materials making up the disposable pull-on diaper 1B of the second embodiment are described below.
The materials used to make the disposable pull-on diaper 1B of the second embodiment are the same as those for the disposable pull-on diaper 1A of the first embodiment.

The disposable pull-on diaper 1B according to the second embodiment of the invention produces the following advantages.
The advantages of the diaper 1B of the second embodiment will be explained with respect to the differences from the advantages of the diaper 1A of the first embodiment. The advantages of the diaper 1B are otherwise the same as those of the diaper 1A so that the description about the advantages of the disposable diaper 1A of the first embodiment applies as appropriate.

The absorbent assembly 3 of the diaper 1B of the second embodiment is bonded on its vertical (direction Y) front end portion 3a to the front panel 2A with an adhesive 7 applied in the shape of a T and on its vertical (direction Y) rear end portion 3b to the rear panel 2B with an adhesive 7 applied in the shape of an inverted T. Since each of the front panel 2A and the rear panel 2B is fixed to the absorbent assembly 3 in a laterally (direction X) middle part of the elasticized extension region G3, the diaper 1B has a good appearance. Since, similarly to the first embodiment, the absorbent assembly 3 is not bonded to the front and the rear panel 2A and 2B in the region where each of the longitudinal (direction Y) end portions 34a and 34b of each fiber-free region 34 overlaps the front and the rear panel 2A and 2B, the absorbent member 33 easily bends over the whole length of the crotch portion C to provide further improved fit to the wearer's skin.

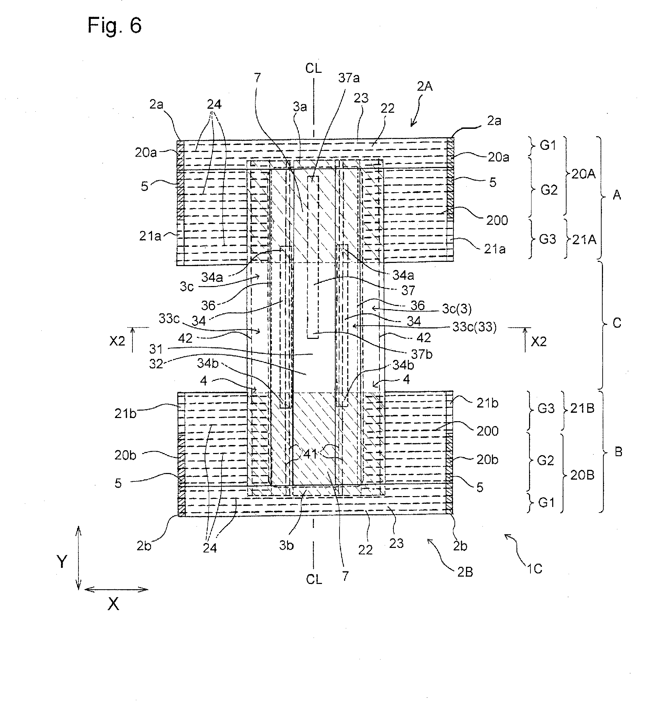

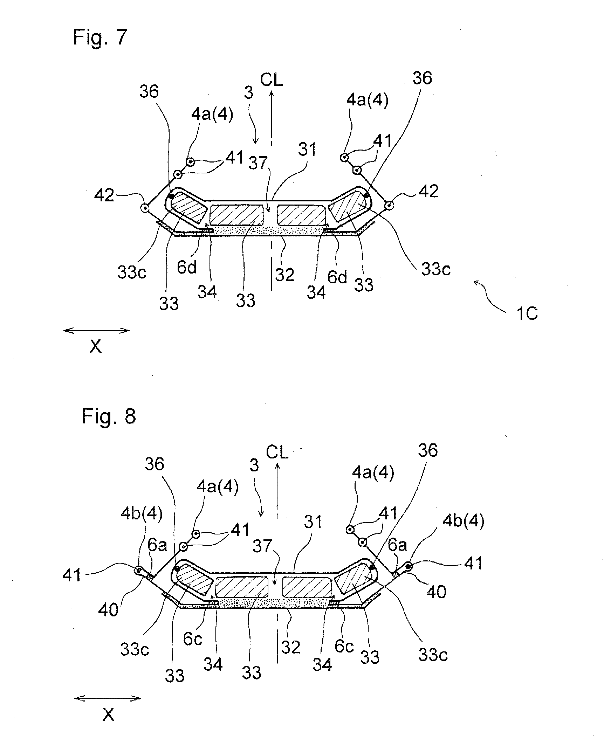

A disposable pull-on diaper of a third embodiment of the invention will be described by way of Figs. 6 and 7.
The disposable pull-on diaper 1C according to the third embodiment (hereinafter "diaper 1C") will be described with respect to the differences from the diaper 1A of the first embodiment. The diaper 1C is otherwise equal to the diaper 1A so that the description of the diaper 1A will be applied as appropriate for the rest.

The absorbent assembly 3 of the diaper 1C of the third embodiment is bonded on its vertical (direction Y) end portion 3 a to the front panel 2A with an adhesive 7 and on its vertical (direction Y) rear end portion 3b to the rear panel 2B with an adhesive 7. In detail, as shown in Fig. 6, in the diaper 1C of the third embodiment, the front end portion 3a of the absorbent assembly 3 and a laterally (direction X) middle part of the front panel 2A are bonded to each other with the adhesive 7, the adhesive 7 being applied solid between the lower (direction Y) edge of the elasticized extension region G3 of the front panel 2A and the front edge of the front end portion 3a of the absorbent assembly 3 and between the opposing distal edges of the lateral side portions 3c, 3c of the absorbent assembly 3. Similarly, as shown in Fig. 6, the rear end portion 3b of the absorbent assembly 3 and a laterally (direction X) middle part of the rear panel 2B are bonded to each other with the adhesive 7, the adhesive 7 being applied solid between the lower edge of the elasticized extension region G3 of the rear panel 2B and the rear edge of the rear end portion 3b of the absorbent assembly 3 and between the opposing distal edges of the lateral side portions 3c, 3c of the absorbent assembly 3.

As shown in Fig. 6, the absorbent member 33 used in the diaper 1C of the third embodiment is structurally different from that in the diaper 1A. Specifically, as shown in Fig. 6, the absorbent member 33 of the diaper 1C is a single-layer structure having only a vertically (direction Y) elongated rectangular lower absorbent layer and having no high basis weight region 35 (upper absorbent layer). The absorbent member 33 of the diaper 1C has a pair of fiber-free regions 34 extending along the respective lateral side portions of the absorbent member 33 such that the vertical front end portion 34a of each fiber-free region 34 overlaps the front panel extension portion 21 A and that the vertical rear end portion 34b of each fiber-free region 34 overlaps the rear panel extension portion 21 B similarly to the diaper 1A. The absorbent member 33 of the diaper 1C additionally has a central fiber-free region 37 at the middle between the pair of fiber-free regions 34. The central fiber-free region 37 extends with its vertical (direction Y) front end portion 37a on the side of the front portion A reaching the front panel main portion and terminating in the elasticized below-waist region G2 of the front panel A. The front end portion 37a of the central fiber-free region 37 extends beyond the positions of the vertical front end portions 34a, 34a of the pair of fiber-free regions 34, 34. The rear end portion 37b of the central fiber-free region 37 on the side of the rear portion B is located at almost the middle between the position of the vertical front end portion 34a of each fiber-free region 34b and the position of the vertical rear end portion 34b of each fiber-free region 34b.

As shown in Fig. 6, the absorbent member 33 of the diaper 1C of the third embodiment has a region in which the central fiber-free region 37 and the pair of fire-free regions 34. 34 are in juxtaposition in the lateral direction (direction X). This region will hereinafter be referred to as a juxtaposition region. The juxtaposition region allows the absorbent member 33 to bend into a "W" configuration when worn, with the central fiber-free region 37 as the top of a mountain and each fiber-free region 34, 34 as the bottom of valleys.
Because the absorbent member 33 is bendable in the crotch portion C into a "W" configuration, it is able to flexibly change its shape in response to the pressure applied by the wearer's legs so that the pressure applied to the legs and the resultant feel of resistance, discomfort, or the like felt by the legs will be reduced.

The part of the absorbent assembly 3 having the central bending-inducing region 37 easily comes into contact with the point of urine discharge of a wearer so that discharged urine is smoothly wicked into the absorbent member 33. Therefore, inconveniences will be prevented from occurring, such as urine's running on the skin or the topsheet and leaking from the front side of the diaper or urine's running rearward and being mixed with feces to cause the feces to stick to the skin.

To further ensure the above described effects, it is preferred that the vertical front end portion 37a of the central fiber-free region 37 be beyond the front end of each fiber-free region 34 by a distance of 5 to 40 cm, more preferably 10 to 30 cm.

In the diaper 1C of the third embodiment, as earlier described, the central fiber-free region 37 has its vertical (direction Y) rear end portion 37b located at almost the middle between the positions of the vertical front end portion 34a and the vertical rear end portion 34b of each fiber-free region 34. In other words, the vertical rear end portion 34b of each fiber-free region 34 extends beyond the vertical rear end portion 37b of the central fiber-free region 37 in the rear portion B side of the absorbent member 33 Because of this configuration, the part of the absorbent member 33 closer to the rear end of the rear portion B than the juxtaposition region bends along the pair of fiber-free regions 34, 34 without bending along the central fiber-free region 37 while the disper is worn. As a result, the absorbent member 33 forms a dished shape concave toward the skin around the exit point of feces (i.e., the anus), which is rearward of the juxtaposition region, whereby there is formed a pocket concave toward the skin and capable of holding discharged feces between the wearer's skin and the absorbent assembly 3. Discharged urine, on the other hand, is quickly wicked into the absorbent member 33 and led toward the side of the front portion A by the configuration of the juxtaposition region and the central fiber-free region 37 extending beyond the juxtaposition region toward the front. Therefore, urine is less liable to mix with feces.
Thus, inconveniences due to feces' adhering the skin, such as wearer discomfort and the troublesome work to wipe off the fecal material from the skin surface, are avoided or greatly reduced. Excellent protection against feces leakage is also provided.

To further ensure the effects discussed above, it is preferred that the rear end portion 34b of each fiber-free region 34 be located rearward beyond the end portion 37b of the central fiber-free region 37 by a distance of 20 to 120 cm, more preferably 40 to 100 cm.

As shown in Figs. 6 and 7, the diaper 1C of the third embodiment has an elastic member 36 for the absorbent member disposed in its stretched state in the vertical direction (direction Y) along each lateral side edge portion 33c of the absorbent member 33. The elastic member 36 is disposed in its stretched state to vertically (direction Y) extend along the edge of each lateral side edge portion 33c of the absorbent member 33 and terminate in the front panel 2A and the rear panel 2B.

In the diaper 1C of the third embodiment, the standing gather 4 provided in each lateral side portion 3c of the absorbent assembly 3 has only a first standing gather 4a that extends laterally (direction X) inward from the lateral side portion 3c of the absorbent assembly 3 as shown in Figs. 6 and 7. The first standing gather 4a is formed of a standing gather-forming sheet 40 and a plurality of elastic members 41 and a midway elastic member 42 disposed in their stretched state in the vertical direction (direction Y in a flat-out, uncontracted state of the pull-on absorbent article) of the standing gather-forming sheet 40. Specifically, in the diaper 1C of the third embodiment, the standing gather-forming sheet 40 is a single sheet, which is folded in half into a two-ply sheet along a folding line corresponding to the tip of the free edge of the first standing gather 4a. The plurality of elastic members 41 are fixed in their stretched state in the vertical direction (direction Y) with an adhesive between the two plies of the folded sheet to make the first standing gather 4a as shown in Fig. 7. The midway elastic member 42 is disposed between the two plies and at a position slightly outward from the lateral (direction X) side edge of the absorbent member 33 in its stretched state in the vertical direction (direction Y) and fixed with an adhesive to form the first standing gather 4a.

In the diaper 1C of the third embodiment, the topsheet 31 is larger than the backsheet 32 in the lateral direction (direction X). The portion of the topsheet 31 extending laterally (direction X) outward from each lateral side edge portion 33c of the absorbent member 33 is folded over the backsheet side of the absorbent member 33. As shown in Fig. 7, the ends of the above described two plies of the standing gather-forming sheet 40 are inserted between the part of the topsheet 31 folded over the backsheet side of the absorbent member 33 and the backsheet 32 and bonded thereto at a fourth linear bond 6d formed at the position of the fiber-free region 34 of the absorbent member 33. The thus formed first standing gather 4a rises by the contraction of the plurality of elastic members 41 and the midway elastic member 42 toward the skin of a wearer and laterally (direction X) inward (toward the centerline CL). To ensure rising of the standing gather, the position of the fourth linear bond 6d is preferably right under the fiber-free region 34 of the absorbent member 33 or away from that position toward the centerline CL.

The fourth linear bond 6d extends along each lateral side portion of the rectangular absorbent assembly 3 over the whole length of the absorbent assembly 3 in the vertical direction (direction Y). The fourth linear bond 6d is preferably a continuous straight line but may be a dotted line. The fourth linear bond 6d can be formed by various known methods, such as heat sealing, ultrasonic sealing, high frequency sealing, or application of an adhesive.

In the diaper 1C of the third embodiment, the plurality of elastic members 41 and the midway elastic member 42 forming the first standing gather 4a are disposed so as to exert their contractive force between the front end portion 34a and the rear end portion 34b of each fiber-free region 34 of the absorbent member 33. Providing the plurality of elastic members 41 and the midway elastic member 42 to form the first standing gather 4a in such an arrangement helps the absorbent member 33 of the absorbent assembly 3 to form an outward convex pocket while the diaper is worn.

Materials making up the disposable pull-on diaper 1C of the third embodiment will then be described.
The elastic members 36 disposed on the absorbent member 33 and the midway elastic members 42 forming the first standing gather 4a are the same as the elastic members 41 used in the standing gathers 4 of the disposable pull-on disper 1A of the first embodiment. Materials making the other members are also the same as those used in the diaper 1A of the first embodiment.

The disposable pull-on diaper 1C according to the third embodiment of the invention produces the following advantages.
The advantages of the diaper 1C of the third embodiment will be explained with respect to the differences from the advantages of the diaper 1A of the first embodiment. The advantages and effects of the diaper 1C are otherwise the same as those of the diaper 1A so that the description about the advantages and effects of the disposable diaper 1A of the first embodiment applies as appropriate.

The diaper 1C of the third embodiment has the elastic member 36 for the absorbent member in each lateral side edge portion 33c of the absorbent member 33 as shown in Fig. 7. The absorbent member 33 of the diaper 1C of the third embodiment has the central fiber-free region 37 as well as the pair of fiber-free regions 34, 34 as shown in Figs. 6 and 7. Therefore, both the lateral side edge portions 33c, 33c of the absorbent member 33 having the elastic members 36 easily rise, and the absorbent member 33 easily bends in the crotch portion C along the fiber-free regions 34 to form an outward convex pocket.
Because both the lateral side edge portions 33c, 33c of the absorbent member 33 are not restrained by the first standing gathers 4a as shown in Fig. 7, the rise of the lateral side portions 33c, 33c of the absorbent member 33 in the crotch portion C is not impeded even when, as shown in Fig. 6, the absorbent assembly 3 is fixed with the adhesive 7 to substantially the entire area of the regions where it overlaps the front panel 2A and the rear panel 2B. From the viewpoint of appearance as a garment, each of the front panel extension portion 21A of the front panel 2A and the rear panel extension portion 21B of the rear panel 2B is preferably bonded to the absorbent member 33. This is particularly preferred for the rear panel extension portion 21B to prevent exposure of the wearer's buttocks in the rear.

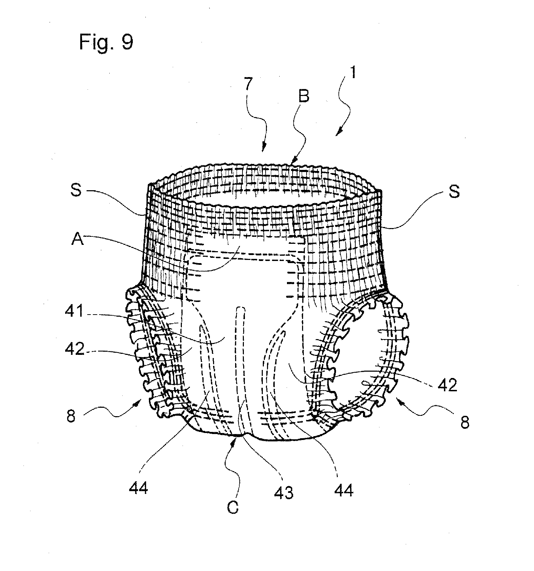

A disposable pull-on diaper according to a fourth embodiment of the invention will be described by way of Figs. 9 through 11.
The disposable pull-on diaper 1D according to the fourth embodiment (hereinafter "diaper 1 D") will be described with respect to the differences from the diaper 1A of the first embodiment. The diaper 1D is otherwise equal to the diaper 1A so that the description of the diaper 1A will be applied as appropriate for the rest.

The diaper 1D is of pull-on type, including, as shown in Figs. 9 through 11, an absorbent assembly 5' and an outer cover 10' located on the non-skin facing side of the absorbent assembly 5' and having the absorbent assembly 5' fixed thereto. The absorbent assembly 5' includes a liquid permeable topsheet 2', a liquid impermeable or water repellent backsheet 3', and a vertically oblong absorbent member 4' interposed between the sheets 2' and 3'.

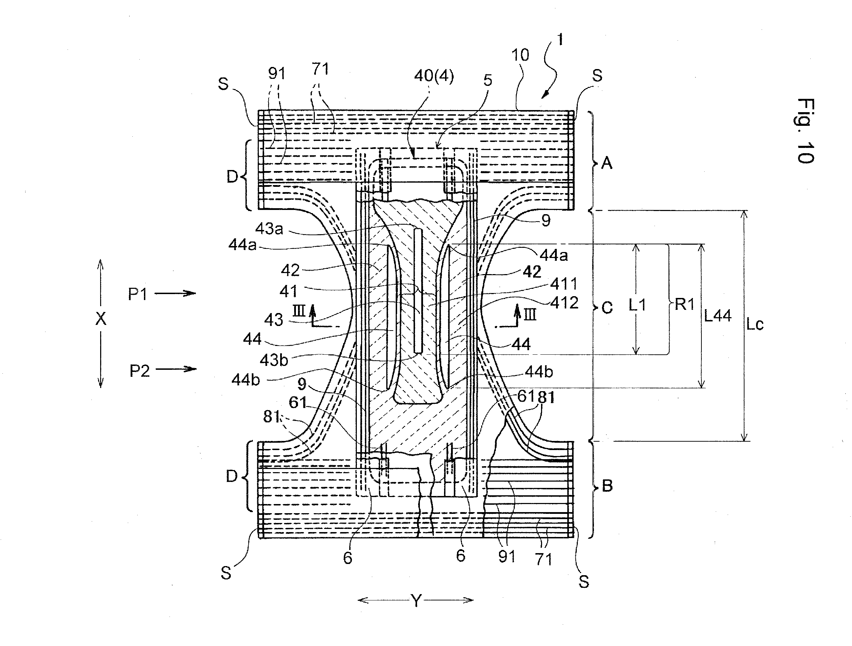

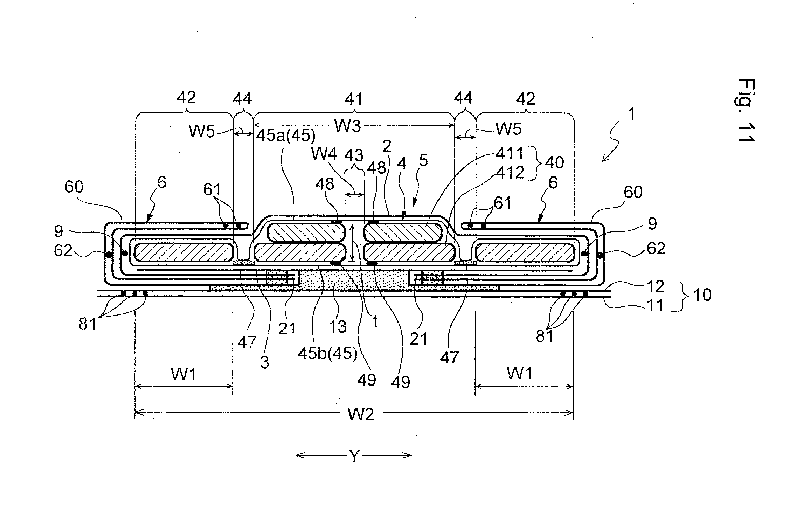

The absorbent member 4' includes a vertically oblong absorbent core 40' containing an absorbent polymer and a core wrap sheet 45' covering the upper and the lower side of the absorbent core 40'. As shown in Fig. 11, the absorbent core 40' of the present embodiment wraps the entire transverse cross-sectional circumference of the absorbent core 40' comprising the upper and the lower surface and both lateral side faces of the absorbent core 40' with the core wrap sheet 45'.
As used for a disposable diaper and an absorbent core thereof, the term "the skin-facing side" or "the upper side" means the side facing the skin of a wearer while the diaper is worn, and the term "the non-skin-facing side" or "the lower side" means the side facing opposite to the wearer's skin while the diaper is worn.
As shown in Figs. 9 and 10, the diaper 1D has a front portion A, a crotch portion B, and a rear portion C, to be worn about the front side, the crotch, and the rear side, respectively, of a wearer. The outer cover 10' has its opposite side edge portions in the front portion A joined to those in the rear portion B to form a pair of side seals S, S, a waist opening 7', and a pair of leg openings 8', 8'.

The front portion A and the rear portion B of the disposable pull-on diaper are each defined to be a portion having the side seal S along both lateral side edges opposite in the transverse direction of the diaper (direction X in Fig. 10). The longitudinal direction of the diaper is the direction extending from the front portion A through the crotch portion C to the rear portion B or the opposite direction, which is the direction Y in Fig. 10. In the diaper 1D of the present embodiment, all of the longitudinal direction of the diaper 1D, the longitudinal direction of the absorbent member 4', the longitudinal direction of the absorbent core 40', and the longitudinal direction of a hereinafter described central absorbent portion 41' are the direction Y in Fig. 10.

As shown in Figs. 10 and 11, the absorbent core 40' in the diaper 1D has, in the crotch portion C, a central absorbent portion 41' extending in the longitudinal direction of the absorbent core 40' and a pair of side absorbent portions 42', 42' located on both sides of the central absorbent portion 41'. In detail, as shown in Fig. 10, the absorbent core 40' is composed of an upper absorbent layer 411 having a generally T-shape in a plan view with an increased width in its front portion side and a rectangular lower absorbent layer 412 larger than the upper absorbent layer 411 in both the longitudinal and the transverse direction.
As shown in Fig. 11, the lower absorbent layer 412 has a pair of through-holes as fiber-free regions 44', 44'. The side absorbent portions 42', 42' are portions located laterally (in the direction X) outward from the fiber-free regions 44', 44', and the central absorbent portion 41' is a portion located between the fiber-free regions 44', 44'.
The upper absorbent layer 411 extends from the crotch portion C to the front portion A. The lower absorbent layer 412 extends through the crotch portion C and terminates in the front portion A and the rear portion B.

As shown in Figs. 10 and 11, the absorbent core 40' of the diaper 1D has a central fiber-free region 43', where the absorbent core is cut out throughout the thickness, in the laterally middle part of the central absorbent portion 41' as well as the fiber-free regions 44', 44' between the central absorbent portion 41' and both side absorbent portions 42', 42'. Each fiber-free region 44' is located between the corresponding lateral side edge of the upper absorbent layer 411 and the lateral side edge of the absorbent core 40 and closer to the side edge of the upper absorbent layer 411 than to the side edge of the absorbent core 40'. Each fiber-free region 44' extends along each side edge of the central absorbent portion 41'.

The upper absorbent layer 411 and the lower absorbent layer 412 making up the absorbent core 40' of the present embodiment are each formed of a fiber aggregate made of a fibrous material, such as defibrated pulp, with or without a particulate absorbent polymer incorporated therein. The absorbent member 4' is formed by totally covering the upper absorbent layer 411 and the lower absorbent layer 412 with a core wrap sheet 45'. The core wrap sheet may be of various known materials, preferably thin paper (e.g., tissue) or water pervious nonwoven fabric.

The fiber-free region 44' is a region having no materials forming the absorbent core (core-forming material). As shown in Fig. 11, the upper and the lower panel of the core wrap sheet covering the absorbent core 40' are bonded to each other in each fiber-free region 44'. Although it is preferred that there be no core-forming material at all in the fiber-free region 44' so that the core wrap sheet panels may firmly be bonded, presence of a small amount of a core-forming material is acceptable as long as the direct bonding of the core wrap sheet panels with an adhesive is not hindered.
On the other hand, the central fiber-free region 43' is a region of the central absorbent portion 41' where at least part of the upper side thereof is cut out. The central fiber-free region 43' preferably has a depth equal to or greater than the thickness of the upper absorbent layer, more preferably a depth substantially equal to the thickness t of the central absorbent portion 41' at a position adjacent to the central fiber-free region 43' as shown in Fig. 11.

As shown in Fig. 11, the central fiber-free region 43' in the present embodiment is a through-hole piercing the thicknesses of both the upper and the lower absorbent layer 411 and 412. The through-hole piercing the upper and the lower absorbent layer 411 and 412 may be formed by making an upper and a lower absorbent layer each having a through-hole and stacking them with their through-holes aligned with each other; by making an upper and a lower absorbent layer each having no through-hole, cutting a through-hole in each of them, and stacking them with their through-holes aligned with each other; or by making an upper and a lower absorbent layer each having no through-hole, stacking them, and cutting a through-hole piercing the stacked layers.

In the present embodiment, the core wrap sheet panel 45a' overlying the absorbent core 40' is pressed into each fiber-free region 44', and the pressed-in part of the panel 45a' is bonded to the core wrap sheet panel 45b' underlying the absorbent core 40' via an adhesive 47'. The adhesive 47' may be applied either continuously or discontinuously in the longitudinal direction of the fiber-free region 44'. While the adhesive may be applied solid to the entire adherend area corresponding to the fiber-free region 44', it is preferably applied in a regular pattern over the whole length of the fiber-free region 44'. For example, the adhesive 47' may be applied to the core wrap sheet panel 45a' or 45b' in a pattern, such as a spiral, a continuous wave, a zig-zag, or a continuous omega pattern, with a width equal to or larger than the width of the fiber-free region 44' along the longitudinal direction of the absorbent core 40'. The absorbent core 40' is placed on or under the core wrap sheet with its fiber-free regions 44' in register with the applied adhesive, and the upper and the lower core wrap sheet panels are bonded to each other via the adhesive. The width of the applied adhesive 47' may be smaller than the width of the fiber-free region 44'. For example, the adhesive may be applied to form spaced away adhesive layers within the width of the fiber-free region 44' each extending in the longitudinal direction of the fiber-free region 44'. The adhesive 47' is preferably applied over a length of at least 50%, more preferably 80% or more, of the whole length L44 of the fiber-free region 44' and over the whole length of the juxtaposition region R1, more preferably over the whole length of the fiber-free region 44'.

On the other hand, the part of the core wrap sheet panel 45a' overlying the absorbent core 40' and corresponding to the central fiber-free region 43' is not bonded to the member lying right under that part of the core wrap sheet panel 45a', i.e., the lower core wrap sheet panel 45b'. It is preferred that the adhesive be absent on the lower side (the absorbent core side) of the part of the core wrap sheet panel 45a' corresponding to the central fiber-free region 43', but the adhesive may be present on that part. Even when there is an adhesive on that part of the upper core wrap sheet panel 45a', a space in which the absorbent polymer or the absorbent core 40' containing the absorbent polymer is allowed to swell upon fluid absorption would be secured unless the adhesive causes adhesion to the part of the member located right thereunder.

When a means is taken to prevent the adhesive from contacting the member located right thereunder, a space allowing the absorbent polymer or the absorbent core 40' containing the absorbent polymer to swell upon fluid absorption will be secured more certainly between the lower side of the core wrap sheet panel 45a' and the member located right thereunder.
As will be described with respect to a preferred method of production, such a means is exemplified by applying a tension to the core wrap sheet panel 45a' between the two fiber-free regions 44', 44' when bonding the upper and the lower core wrap sheet panels 45a' and 45b' to each other in the fiber-free regions 44', 44', the tension being applied by using a pressure roller 141 having a pressing projection at the position corresponding to each fiber-free region 44' with no pressing projection at the position corresponding to the central fiber-free region 43'. In the case where an adhesive (e.g., a pressure-sensitive adhesive) is applied as well to the part of the core wrap sheet panel 45a' corresponding to the central fiber-free region 43' or, when the central fiber-free region 43' is a through-hole, to the part of the core wrap sheet panel 45b' corresponding to the central fiber-free region 43', it is desirable that the width W4 of the central fiber-free region 43' is 20 mm or less, more desirably 12 mm or less, so as to prevent the bonding via the adhesive.

It is preferred that the absorbent core 40' be fixed to the underlying core wrap sheet panel 45b' in other than the regions corresponding to the central fiber-free region 43' and the fiber-free regions 44', 44' with an adhesive (not shown) applied in such a regular pattern as recited above, i.e., a spiral, a continuous wave, a zig-zag, or a continuous omega pattern.

In the embodiment shown in Fig. 11, the core wrap sheet panel 45a' overlying the absorbent core 40' is bonded via an adhesive 48' to the absorbent core 40' at positions oppositely neighboring the central fiber-free region 43' in the transverse direction thereof. Similarly, the core wrap sheet panel 45b' underlying the absorbent core 40' is bonded via an adhesive 49' to the absorbent core 40' at opposite positions neighboring the central fiber-free region 43' in the transverse direction thereof. Bonding the absorbent core 40' to the core wrap sheet panels 45a' and/or 45b' at positions oppositely neighboring the central fiber-free region 43' in the transverse direction thereof is effective in preventing every portion of the absorbent core 40' from losing its shape or from deviating from the positional relationship among portions as designed, thereby to secure a space for allowing the absorbent polymer or the absorbent core 40' containing the absorbent polymer to swell upon fluid absorption more certainly. The adhesive 48' and the adhesive 49' are each preferably applied in a regular pattern in the longitudinal direction of each fiber-free region 44'. The adhesive 48' and the adhesive 49' are preferably applied over a length of at least 50%, more preferably 80% or more, of the whole length L44 of the fiber-free region 44' and over the whole length of the juxtaposition region R1, more preferably over the whole length of the fiber-free region 44'. The adhesive 48' and 49' may be applied either solid or patternwise in such a pattern as recited as for the adhesive 47'.

According to the configuration of the diaper 1D of the present embodiment, the central fiber-free region 43' provides a space defined by the absorbent core 40' and the overlying core wrap sheet panel 45a', and the core wrap sheet panel 45a' on the central fiber-free region 43' is not bonded to the member located right thereunder (the core wrap sheet panel 45b'). Therefore, when a fluid is wicked into the central absorbent portion 41' to cause the absorbent polymer present therein to swell, the most of the volumetric increase of the absorbent polymer is absorbed by that space, so that the absorbent polymer is allowed to swell sufficiently without any hindrance.
As a result, the absorptivity of the absorbent polymer is developed to the full, and incorporation of the absorbent polymer effectively leads to improvement on disposable diaper performance.
The bonding of the upper and the lower core wrap sheet panels 45a' and 45b' overlying and underlying, respectively, of the absorbent core in the fiber-free regions 44' guarantees excellent shape retention of the absorbent core 40'. That is, the central absorbent portion 41' and the side absorbent portions 42', 42' are prevented from losing their shape or being misaligned with each other while the diaper is worn or in other situations.

As earlier described, the central absorbent portion 41' allows the absorbent polymer contained therein to fully exert its absorptivity by virtue of the presence of the central fiber-free region 43'. This being taken advantage of, it is preferred that absorbent polymer be incorporated in a higher basis weight (amount per unit area) in the central absorbent portion 41' than in the side absorbent portions 42', 42' to achieve efficient improvement on the performance of the disposable diaper expected of the incorporation of an absorbent polymer. From the same viewpoint, in the case where the central absorbent portion 41' has a multi-layered (or dual-layered) structure having the upper absorbent layer 411 and the lower absorbent layer 412, the absorbent polymer is preferably incorporated in the upper absorbent layer 411 in a higher basis weight than in the lower absorbent layer 412. In the case of a multi-layered structure having three or more layers, the absorbent polymer is preferably incorporated into the uppermost absorbent layer in a higher basis weight than any other absorbent layer.

The central fiber-free region 43' according to the present embodiment also functions to accelerate spread of a fluid in the longitudinal direction of the absorbent core 40' and to improve transverse bending properties of the absorbent core 40'.
The fiber-free regions 44' according to the present embodiment function to accelerate spread of a fluid in the longitudinal direction of the absorbent core 40' and to improve transverse bending properties of the absorbent core 40'.

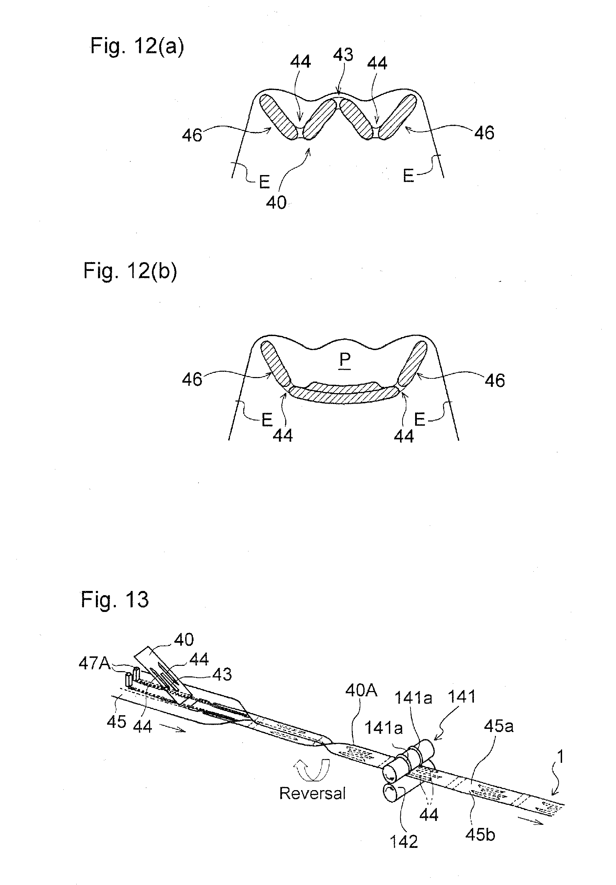

As shown in Fig. 10, the absorbent member 4' in the diaper 1D of the present embodiment has, in its absorbent core 40' in the crotch portion C, a region R1 wherein the central fiber-free region 43' and the pair of fiber-free regions 44', 44' are in juxtaposition in the transverse direction of the absorbent core 40' (hereinafter also called "juxtaposition region R1").
Because of the presence of the juxtaposition region R1 in the crotch portion C, the part of the absorbent core 40' located in the juxtaposition region R1 bends into a "W" configuration when worn, with the central fiber-free region 43' as the top of a mountain and each fiber-free region 44' as the bottom of a valley, as shown in Fig. 12(a).
Since the absorbent core 40' is bendable into a W-configuration in the crotch portion C, the absorbent core 40' can flexibly change its shape in response to the pressure applied by the wearer's legs E so that the pressure applied to the legs and the resultant resistance, discomfort, or the like felt by the legs will be reduced. Moreover, side portions 46' of the absorbent core 40' outboard of the fiber-free regions 44' rise to have their distal edge located along the wearer's skin, preferably the groins, thereby to provide good protection against leakage.

The part of the absorbent assembly 5' having the central fiber-free region 43' easily comes into contact with the wearer's exit point of urine so that urine is smoothly wicked into the absorbent member 4' or the absorbent core 40'. The urine having been acquired by the absorbent member 4' or the absorbent core 40' spreads forward and rearward so that the absorption capacity of the portions of the absorbent core 40' located in the front and the rear of the juxtaposition region R1 will be utilized effectively as well. As a result, the amount of urine absorbable by the diaper 1D without leakage increases, and urine leakage hardly occurs even during long-time use of the diaper or with a large amount of urination. Since the urine spreads as captured by the absorbent member 4' or the absorbent core 40', the problems due to the contact of the skin with urine, such as an uncomfortable wetness sensation and diaper rash, will be reduced.

The part of the absorbent core 40' closer to the rear end of the rear portion B than the juxtaposition region R1 bends along the fiber-free regions 44', 44' as shown in Fig. 12(b) while the diaper is worn. As a result, the absorbent core 40' forms a dished shape concave toward the skin around the exit point of feces (i.e., the anus), which is rearward of the juxtaposition region R1, whereby there is formed a pocket P concave up and capable of holding discharged feces between the wearer's skin and the absorbent assembly. Thus, inconveniences due to feces' adhering the skin, such as wearer discomfort and the troublesome work to wipe off the fecal material from the skin surface, are avoided or greatly reduced. Excellent protection against feces leakage is also provided.

To further ensure one or more of the above described effects and advantages, it is preferred that the length L1' (see Fig. 10) of the juxtaposition region R1 in the longitudinal direction of the absorbent core 40' be in the range of from 20% to 80%, more preferably 20% to 60%, even more preferably 20% to 50%, of the length Lc (see Fig. 10) of the crotch portion C in the diaper longitudinal direction.
It is preferred that the width W1' (see Fig. 11) of the side absorbent portion 42' be 5% to 30%, more preferably 10% to 25%, of the width W2' (see Fig. 11) of the absorbent core 40'. The width W3 (see Fig. 11) of the central absorbent portion 41' is preferably 20% to 70%, more preferably 25% to 50%, of the width W2' of the absorbent core 40'. The width W4 of the central fiber-free region 43' is preferably 1 to 25 mm, more preferably 2 to 15 mm, and is preferably, in terms of relation to the width W3 of the central absorbent portion 41', 3% to 30%, more preferably 5% to 25%, of W3 of the central absorbent portion 41'. The width W5 of the fiber-free region 44' is preferably 1 to 35 mm, more preferably 5 to 25 mm, and is preferably, in terms of relation to the width W2' of the absorbent core 40', 1% to 30%, more preferably 5% to 20%, of W2' of the absorbent core 40'.

As shown in Figs. 10 and 11, the diaper 1D of the present embodiment has an elastic member 9' disposed along each lateral side edge of the absorbent core 40' to raise the side portions 46' of the absorbent core 40'. The elastic member 9' is disposed in its stretched state along the interior and/or the exterior side of the core wrap sheet 45' covering the absorbent core 40' and fixed at both ends thereof in the front portion A and the rear portion B.
A pair of anti-leakage cuffs 6', 6' are formed along both lateral sides of the absorbent assembly 5'. Each anti-leakage cuff 6' includes a cuff-forming sheet 60' bonded to the absorbent assembly 5', cuff-forming elastic members 61' fixed in their stretched state near along the free edge of the cuff-forming sheet 60', and a midway elastic member 62' fixed in its stretched state between the fixed edge and the free edge of the anti-leakage cuff 6'.
The cuff-forming sheet 60' used in the embodiment shown in Fig. 11 is a two-ply sheet formed by folding an oblong water-repellent sheet with a prescribed width in half lengthwise, the facing two plies being bonded to each other with a hot melt adhesive, partial heat or ultrasonic sealing, or a like means. The elastic members 61' and 62' are fixed in their stretched state between the two plies.

The topsheet 2' covers the upper side (skin facing side) of the absorbent core 40', with each side portions 21' thereof being fixed to the backsheet 3' and the cuff-forming sheet 60' on the lower side (non-skin-facing side) of the absorbent core 40' by a known bonding means, such as heat sealing or an adhesive. The absorbent assembly 5' is bonded to the outer cover 10' with an adhesive 13' in its laterally middle portion.

As shown in Figs. 10 and 11, the outer cover 10' is composed of two outer cover-forming sheets 11', 12' and elastic members for specific parts fixed in their stretched state between these two sheets. Specifically, there are waist elastic members 71' forming waist gathers along the waist opening 7', leg elastic members 81' forming leg gathers along the leg openings 8', and below-waist elastic members 91' forming below-waist gathers on two spaced apart lateral sides of the below-waist portion D (the region between a position 20 mm below the edge of the waist opening 7' and the upper end of each leg opening 8') fixedly bonded in their stretched state between the two outer cover-forming sheets 11, 12 by any bonding means, such as a hot melt adhesive.
The outer cover-forming sheets 11' and 12' are each formed of nonwoven fabric. At least one of these sheets is folded back along the circumference edge of the waist opening 7', and the folded-back panel is fixed to the skin facing side of the front and the vertical rear end portion of the absorbent assembly 5.

In order for the absorbent core 40' to bend into a W-configuration in the juxtaposition region R1 as shown in Fig. 12(a) and/or into a dish-shaped configuration in the region rearward of the juxtaposition region R1 as shown in Fig. 12(b), it is preferred that elastic members effective in pulling up or pushing up the side portions 46' of the absorbent core 40' be disposed along both sides of the absorbent assembly 5', the anti-leakage cuffs 6' provided to overlap both side portions of the absorbent assembly 5', and so on like the elastic members 9', 61', and 62'.
It is to be noted that, even if there are no elastic members disposed along the longitudinal direction of the absorbent core 40', the absorbent core 40' is deformable into the shape shown in Figs. 12(a) or 12(b) by a vertical force of grasping and pulling up the laterally middle part of the front portion A when the diaper is fitted onto a wearer or a sideways force applied by the wearer's thighs to the crotch portion C while the disper is worn.

Materials making up the diaper 1D will be described.
Any various materials that have been used in each member of conventional disposable diapers may be used to make the corresponding members of the diaper 1D. For example, the topsheet 2' may be formed of water-wettable and liquid permeable nonwoven fabric or perforated resin film, and the backsheet 3' may be formed of moisture permeable or impermeable resin film or a laminate of such resin film and nonwoven fabric.
Fibers constituting the fiber aggregate forming the absorbent core 40' include hydrophilic fibers, such as pulp fiber, rayon fiber, cotton fiber, and cellulose acetate fiber, polyolefin fibers, such as polyethylene and polypropylene, and polycondensation fibers, such as polyesters and polyamides. The fiber aggregate is preferably an airlaid fiber aggregate prepared by accumulating a fibrous material carried in an air stream, either alone or as mixed with a functional material, in a recess of prescribed shape by suction. A functional material, if used, may be held between two fiber aggregates or among fibers in a mixed airlaid fiber aggregate. Two or more kinds of fibers or two or more functional materials, such as absorbent polymers, may be used in combination. Examples of the particulate absorbent polymer include sodium polyacrylate, acrylic acid-vinyl alcohol copolymers, crosslinked sodium polyacrylate, a starch-acrylic acid graft copolymer, an isobutylene-maleic anhydride copolymer and a saponification product thereof, and polyaspartic acid.

The anti-leakage cuff-forming sheet 60' may be, for example, composite nonwoven fabric having a multi-layer structure (e.g., spun-bonded/melt-blown/spun-bonded), spun-bonded nonwoven fabric, heat-bonded nonwoven fabric, or air-through nonwoven fabric. The elastic member to be disposed in various parts or members is preferably an elastic thread (e.g., rubber thread) or an elastic tape with a certain width (e.g., rubber tape), particularly an elastic thread. Materials of the elastic member to be disposed in various parts or members include natural rubber, synthetic rubbers, e.g., styrene-butadiene rubber, butadiene rubber, isoprene rubber, and neoprene rubber, EVA, stretch polyolefins, and urethane rubber.

The above mentioned diaper 1D is preferably produced by a method described below.
The method includes the steps of making an absorbent core 40' composed of a central absorbent portion 41' having a central fiber-free region 43' and a pair of side absorbent portions 42', 42' having respective fiber-free regions 44' along the central absorbent portion 41' (unshown step), applying an adhesive 47' to parts of a core wrap sheet 45' corresponding to the fiber-free regions 44', and pressing the absorbent core 40' having the core wrap sheet 45' on the upper and the lower side thereof using a pressure roller 141 having a pressing projection 141 a at the position corresponding to each fiber-free region 44' with no pressing projection at the position corresponding to the central fiber-free region 43', thereby to press one or both of the upper and the lower panels of the core wrap sheet 45' into the fiber-free regions 44' to bond the upper and the lower core wrap sheet panels 45a' and 45b' to each other.

The absorbent core 40' is preferably made using a fiber airlaying apparatus having, for example, two airlaying drums each having a recess of prescribed shape in which a fibrous material transported by air is deposited by suction. A lower absorbent layer 412 having through-holes providing a central fiber-free region 43' and fiber-free regions 44', 44' is formed on one of the airlaying drums, and an upper absorbent layer 411 having a through-hole providing the central fiber-free region 43' is formed on the other airlaying drum. The absorbent layers 411 and 412 are stacked to make the absorbent core 40' having a through-hole piercing the two absorbent layers as a central fiber-free region 43'. The adhesive 47' can be applied to the parts of the core wrap sheet 45' corresponding to the fiber-free regions 44' by the use of any known adhesive applicator 47A'.

Bonding the upper and the lower core wrap sheet panels 45a' and 45b' in the fiber-free regions 44' is achieved by pressing using a combination of the above described pressure roller 141 and either one of a smooth roller 142 and a second roller 142 having similar projections at the positions corresponding to the pressing portions 141a of the pressure roller 141. The projections as the pressing portions 141a may be continuous around the entire circumference of the pressure roller 141 or over a certain length in the circumferential direction of the pressure roller 141. The diaper 1D can be produced in the same manner as for the production of conventional disposable pull-on diapers except for the making of the absorbent member 4'. For example, the method described in JP 2006-247363A may be followed to advantage.

When the parts of the core wrap sheet panel 45a' corresponding to the fiber-free regions 44' are pressed in and bonded to the core wrap sheet panel 45b', a tension is applied to the core wrap sheet panel 45a' between the two fiber-free regions 44', 44'. As a result, a space allowing the absorbent polymer or the absorbent core containing the absorbent polymer to easily swell upon fluid absorption will be secured more certainly between the upper surface of the absorbent core 40' and the core wrap sheet panel 45a'.

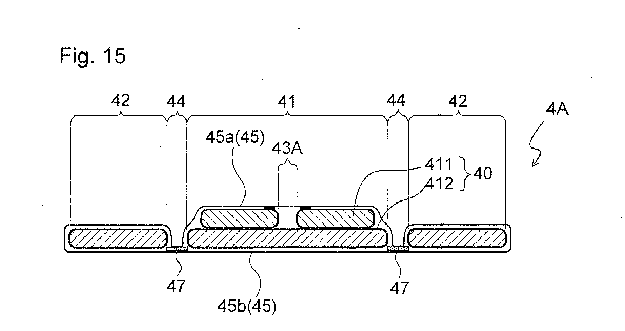

Another embodiment of the invention will be described. Fig. 15 is a transverse cross-section of an absorbent member 4A' used in a disposable diaper according to another embodiment of the invention. The description of this embodiment will be confined to the differences from the diaper 1D already discussed. For the rest, the description of the diaper 1D including the preferred mode will be applied as appropriate.

The absorbent core 40' of the absorbent member 4A' shown in Fig. 15 is composed of an upper absorbent layer 411 having a through-hole providing a central fiber-free region 43'A and a lower absorbent layer 412 having no through-hole aligned with the through-hole of the upper absorbent layer 411. Like this configuration, the central fiber-free region may be a through-hole (fiber-free region) formed through only the upper absorbent layer 411 that forms the central absorbent portion 41' together with the lower absorbent layer 412.

In the embodiment shown in Fig. 15, too, the core wrap sheet panel 45a' overlying the absorbent core 40' is pressed inside each fiber-free region 44', and the pressed-in part is bonded to the core wrap sheet panel 45b' underlying the absorbent core 40' via an adhesive 47'. On the other hand, the part of the core wrap sheet panel 45a' overlying the central fiber-free region 43'A of the absorbent core 40' is not bonded to the upper side of the lower absorbent layer 412 that is a member located right thereunder.

In the embodiment shown in Fig. 15, too, when a fluid is wicked into the central absorbent portion 41' to cause the absorbent polymer present therein to swell, the presence of the central fiber-free region 43'A allows the absorbent polymer to swell sufficiently without encountering a hindrance. As a result, the absorptivity of the absorbent polymer is developed to the full, and incorporation of the absorbent polymer effectively leads to improvement on disposable diaper performance. The bonding of the upper and the lower core wrap sheet panel 45a' and 45b' overlying and underlying, respectively, of the absorbent core guarantees excellent shape retention of the absorbent core. That is, the central absorbent portion 41' and the side absorbent portions 42', 42' are prevented from losing their shape or being misaligned with each other during use.

The pull-on absorbent article of the invention is not limited to the above discussed disposable pull-on diapers according to the first to fourth embodiments, and various changes and modifications can be made therein as described below. Every constituent feature of the disposable pull-on diapers according to the first to fourth embodiments may be implemented in appropriate combination unless the effect of the invention is not impaired.

While in the disposable pull-on diapers 1A, 1B, and 1C of the first to the third embodiment shown in Figs. 2, 5, and 6, the front panel 2A has the front panel main portion 20A and the front panel extension portion 21A extending downward from the lower edge 200 of the front panel main portion 20A, and the rear panel 2B has the rear panel main portion 20B and the rear panel extension portion 21B, one or both of the front panel extension portion 21A and the rear panel extension portion 21B may be dispensed with.

In the disposable pull-on diaper 1C according to the third embodiment, the standing gather 4 may be composed of a first standing gather 4a extending laterally (direction X) inward from the lateral side portion 3c of the absorbent assembly 3 and a second standing gather 4b extending laterally (direction X) outward from the side portion 3c of the absorbent assembly 3 as shown in Fig. 8. Each of the first and the second standing gather 4a and 4b is formed of a standing gather-forming sheet 40 and a plurality of elastic members 41 disposed in their stretched state in the vertical direction (direction Y in a flat-out, uncontracted state of the pull-on absorbent article) of the standing gather-forming sheet 40. As shown in Fig. 8, the standing gather-forming sheet 40 is a single sheet, which is folded in half into a two-ply sheet along a folding line corresponding to the tip of the free edge of the first standing gather 4a. The plurality of elastic members 41 are disposed in their stretched state along the vertical direction (direction Y) between the two plies of the folded sheet and fixed there with an adhesive to make the first standing gather 4a.

The two-ply sheet extends laterally (direction X) outward and is further folded in half along a folding line corresponding to the tip of the free edge of the second standing gather 4b into a four-ply sheet as shown in Fig. 8. The plurality of elastic members 41 are disposed and fixed in their stretched state in the longitudinal direction (direction Y) between the two-ply panels of the four-ply sheet with an adhesive to form the second standing gather 4b'. As shown in Fig. 8, the topsheet 31 is longer than the backsheet 32 in the lateral direction (direction X), and the portion of the topsheet 31 extending outward from each lateral side edge portion 33c of the absorbent member 33 is folded back over the back side of the absorbent member 33. As shown in Fig. 8, the other edge of the two-ply sheet is inserted between the folded-back portion of the topsheet 31 and the backsheet 3 and fixed thereto at near a third linear joint 6c formed at the position of the fiber-free region 34 of the absorbent member 33. The thus formed first standing gather 4a rises by the contraction of the plurality of elastic members 41 toward the skin of a wearer and laterally (direction X) inward (toward the centerline CL). The thus formed second standing gather 4b rise by the contraction of the plurality of elastic members 41 toward the wearer's skin and laterally (direction X) outward (away from the centerline CL). The position of the third linear bond 6c is preferably right under the fiber-free region 34 of the absorbent member 33 or away from that position toward the centerline CL.

The third linear bond 6c extends along each lateral side portion of the rectangular absorbent assembly 3 over the whole length of the absorbent assembly 3 in the vertical direction (direction X). The third linear bond 6c is preferably a continuous straight line but may be a dotted line. The third linear bond 6c can be formed by various known methods, such as heat sealing, ultrasonic sealing, high frequency sealing, or application of an adhesive.

The diaper 1D may be disposable pull-on diaper of separate outer cover type in which the outer cover is separated into a rear side panel adapted to be worn about the rear side of a wearer and a front side panel adapted to be worn about the front side of a wearer, the rear side panel and the front side panel being joined to each other along a pair of side seals into a cylindrical shape, and the absorbent assembly being fixed to bridge the front side panel and the rear side panel. The diaper 1D may also be of flat type having a fastening tape in the rear portion and a landing tape receiving the fastening tape on the outer side of the front portion. In such a flat type disposable diaper, the crotch portion is a portion having a concave cutout in conformity to the contour of a wearer's leg along each lateral side edge thereof, and the rear and the front portion are portions adjacent to and forward and backward, respectively, of the crotch portion.

The core wrap sheet of the diaper 1D may be a single sheet wrapping the entire transverse cross-sectional circumference of the absorbent core or may include two separate sheets, one overlying and the other underlying the absorbent core. In the case when a single core wrap sheet wraps around the absorbent core, the joint (preferably a lap joint) of the opposite edges of the sheet may be on either the upper side or the lower side but is preferably on the lower side of the absorbent core, more preferably between the laterally opposite fiber-free regions 44', 44'. In the case when two wrap sheets are used, it is preferred that both lateral side portions of the underlying sheet be folded over the upper side of the absorbent core and that both lateral side portions of the overlying sheet be bonded to the folded-over portions of the underlying sheet. Otherwise, it is preferred that both lateral side portions of the overlying sheet be folded over the lower side of the absorbent core and that both lateral side portions of the underlying sheet be bonded to the folded-over portions of the overlying sheet.

In the diaper 1D, the central fiber-free region 43' and the pair of fiber-free regions 44', 44' may be through-holes cut in juxtaposition through a single-layered absorbent core each in a vertically elongated shape in a plan view. In this modification, the central absorbent portion 41' and each side absorbent portion 42' may have the same thickness, or the former may be thicker than the latter. In the case of such a single-layered absorbent member, the absorbent member and the core wrap sheet can be bonded to each other according to the embodiments shown in Figs. 11 and 15 or the preferred modes of these embodiments. By so doing, the same effects as by the diaper 1D and the other earlier stated diapers will be obtained to advantage.
In the diaper 1D, the joints formed by the adhesive 48' and/or the adhesive 49' may be dispensed with.

While, in the diaper 1D shown in Fig. 10, the central fiber-free region 43' extends in the longitudinal direction of the absorbent core toward the front portion A beyond the position of the front end 44a' of each fiber-free region 44', the front end 43a' of the central fiber-free region 43' and the front end 44a' of each fiber-free region 44' may be even in the transverse direction, or the fiber-free regions 44' may extend toward the front portion A beyond the position of the front end 43a' of the central fiber-free region 43'.
While, in the diaper 1D shown in Fig. 10, each fiber-free region 44' extends in the longitudinal direction of the absorbent core toward the rear portion B beyond the position of the rear end 43b' of the central fiber-free region 43', the rear ends 43b' and 44b' of the three fiber-free region may be even in the transverse direction, or the central fiber-free regions 43' may extend toward the rear portion B beyond the position of the rear end 44b' of the fiber-free regions 44'.

The diaper 1D may dispense with the elastic members 9'. The elastic members 62' disposed in the anti-leakage cuffs 6', 6' and even the anti-leakage cuffs per se may be dispensed with. Instead, the absorbent assembly 5' may have an elastic member disposed along and outside each lateral side edge of its absorbent core 40' to form a pair of side gathers along each lateral side edge thereof. This elastic member can serve as an elastic member to raise the corresponding side portion of the absorbent core 40'. The absorbent assembly 5' may not have any elastic member disposed in the longitudinal direction of the absorbent core 40'. The central fiber-free region 43' and each side fiber-free region 44' may be configured to cause the absorbent core 40' to bend into a shape other than the shapes shown in Figs. 12(a) and 12(b).

The pull-on absorbent article of the invention may be a pull-on type sanitary napkin as well as a disposable pull-on diaper for children or adults.

### Industrial Applicability

The invention provides a pull-on absorbent article which is of the type wherein the outer cover is separated into a front panel adapted to be worn around the front of a wearer and a rear panel adapted to be worn around the rear of the wearer and yet provides protection against leakage from the absorbent assembly without causing the absorbent assembly to hinder the walking motion of a wearer while worn.

The absorbent core of the diaper according to the invention shows excellent shape retention and is less likely to hinder the swell of the absorbent polymer, thereby allowing the absorbent polymer to fully exhibit its absorptivity

## Claims

1. A pull-on absorbent article comprising a laterally oblong front panel adapted to be worn around a wearer's front side, a laterally oblong rear panel adapted to be worn around the wearer's rear side, an absorbent assembly fixed to the front and the rear panel to bridge them, and a standing gather along each lateral side portion of the absorbent assembly,
the absorbent assembly comprising a vertically oblong absorbent member, the absorbent member having a pair of fiber-free regions extending in its longitudinal direction, each of the fiber-free regions straddling the front panel and the rear panel.

2. The pull-on absorbent article according to claim 1, wherein the front panel has a front panel main portion and a front panel extension portion extending downward from the lower edge of the front panel main portion while worn, the rear panel has a rear panel main portion and a rear panel extension portion extending downward from the lower edge of the rear panel main portion while worn, and each fiber-free region extends with its vertical front end portion overlapping the front panel extension portion and its vertical rear end portion overlapping the rear panel extension portion.

3. The pull-on absorbent article according to claim 2, wherein the front panel and the rear panel each have a plurality of elastic members disposed vertically spacedly in their laterally stretched state,
the elastic members being not disposed in the region where the vertical front end portion of each fiber-free region overlaps the front panel extension portion and the region where the vertical rear end portion of each fiber-free region overlaps the rear panel extension portion.

4. The pull-on absorbent article according to claim 3, wherein the front panel extension portion and the rear panel extension portion each have a plurality of elastic members disposed vertically spacedly in their laterally stretched state.

5. The pull-on absorbent article according to any one of claims 1 to 4, wherein the standing gather comprises a first standing gather extending laterally inward from the lateral side portion of the absorbent assembly and a second standing gather extending laterally outward from the lateral side portion of the absorbent assembly.

6. The pull-on absorbent article according to any one of claims 1 to 5, wherein the absorbent member has an elastic member disposed in its vertically stretched state along each lateral side edge portion thereof.

7. The pull-on absorbent article according to any one of claims 1 to 6, wherein the absorbent member has a high basis weight region, the high basis weight region straddling the front panel extension portion and the rear panel extension portion.

8. The pull-on absorbent article according to claim 7, wherein the absorbent member comprises an absorbent core, the absorbent core comprising an upper absorbent layer and a lower absorbent layer larger than the upper absorbent layer,
the high basis weight region being the result of stacking the upper absorbent layer on the skin facing side of the lower absorbent layer.

9. The pull-on absorbent article according to any one of claims 1 to 8, wherein each fiber-free region has a closed vertical front end portion in the front portion and a non-closed vertical rear end portion in the rear portion.

10. The pull-on absorbent article according to any one of claims 1 to 9, wherein the absorbent member has a central fiber-free region at the middle between the pair of fiber-free regions, the central fiber-free region extending with its front end portion being located beyond the front end portion of each fiber-free region toward the front portion, and each fiber-free region extending with its rear end portion being located beyond the rear end portion of the central fiber-free region toward the rear portion.

11. The pull-on absorbent article according to any one of claims 1 to 10, wherein each of the pair of fiber-free regions is devoid of pulp fiber and a particulate absorbent polymer which constitute the absorbent core of the absorbent member.

12. The pull-on absorbent article according to any one of claims 1 to 10, wherein each of the pair of fiber-free regions contains a total of up to 30 g/m² of pulp fiber and a particulate absorbent polymer which constitute the absorbent core of the absorbent member.

13. The pull-on absorbent article according to claim 1, wherein the absorbent member comprises a vertically oblong absorbent core containing an absorbent polymer and a core wrap sheet covering the upper and the lower surface of the absorbent core,
the absorbent core having a central fiber-free region at the middle between the pair of fiber-free regions,
the part of the core wrap sheet overlying the absorbent core and the part of the core wrap sheet underlying the absorbent core being bonded to each other in the pair of fiber-free regions, and
the part of the core wrap sheet overlying the absorbent core and the member lying right under that part of the core wrap sheet being not bonded to each other in the central fiber-free region.

14. The pull-on absorbent article according to claim 13, wherein the absorbent member has a central absorbent portion extending in the longitudinal direction of the absorbent core and a pair of side absorbent portions each located on each lateral side of the central absorbent portion, and the absorbent polymer is present in the central absorbent portion in a higher basis weight than in each of the side absorbent portions.

15. The pull-on absorbent article according to claim 13 or 14, wherein the absorbent member has a multi-layered structure comprising an upper absorbent layer and a lower absorbent layer, and the absorbent polymer is present in the upper absorbent layer in a higher basis weight than in the lower absorbent layer.
